# EUROPEAN PATENT SPECIFICATION

(11) **EP 2 490 707 B1**
(45) Date of publication and mention of the grant of the patent: **27.07.2016**
(21) Application number: 10765584.7
(22) Date of filing: 12.10.2010
(51) Int. Cl.: A61K 38/16

(54) **MEDICAL UTILITY OF GLYCAN-BINDING PROTEINS AND GLYCANS**
MEDIZINISCHE NÜTZLICHKEIT VON GLYCANBINDENDEN PROTEINEN UND GLYCANEN
UTILITÉ MÉDICALE DES PROTÉINES LIANT LES GLYCANES ET DES GLYCANES

(30) Priority: 21.10.2009 EP 09013283
(43) Date of publication of application: 29.08.2012
(60) Divisional application: 12167453.5
(73) Proprietor: ETH Zurich, 8092 Zurich (CH); UNIVERSITY OF ZURICH, 8006 Zürich (CH)
(72) Inventor: KUNZLER, Markus, CH-5432 Neuenhof (CH); WÄLTI, Martin, CH-8046 Zurich (CH); BUTSCHI, Alex, CH-8340 Hinwil (CH); AEBI, Markus, CH-5430 Wettingen (CH); HENGARTNER, Michael, CH-8057 Zurich (CH)
(74) Representative: Kasche, André
(86) International application number: PCT/EP2010/006228
(87) International publication number: WO 2011/047794

(56) References cited:
- EP-A1- 1 557 172
- WO-A1-2005/092368
- WO-A1-2007/085057
- WO-A2-89/09601
- WO-A2-2005/065017
- WO-A2-2008/142483
- US-A1- 2006 009 378
- GRIFFITTS JOEL S ET AL: "Glycolipids as receptors for Bacillus thuringiensis crystal toxin", SCIENCE (WASHINGTON D C), vol. 307, no. 5711, 11 February 2005 (2005-02-11), pages 922-925, XP002629519, ISSN: 0036-8075
- KOHATSU LUCIANA ET AL: "Galectin-3 induces death of Candida species expressing specific beta-1,2-linked Mannans", JOURNAL OF IMMUNOLOGY, vol. 177, no. 7, October 2006 (2006-10), pages 4718-4726, XP002629520, ISSN: 0022-1767 cited in the application
- ANNA R YOUNG ET AL: "Galectins in parasite infection and allergic inflammation", GLYCOCONJUGATE JOURNAL, KLUWER ACADEMIC PUBLISHERS, BO, vol. 19, no. 7-9, 1 January 2002 (2002-01-01), pages 601-606, XP019206950, ISSN: 1573-4986, DOI: DOI:10.1023/B:GLYC.0000014091.00844.0A
- BUTSCHI ALEX ET AL: "Caenorhabditis elegans N-glycan core beta-galactoside confers sensitivity towards nematotoxic fungal galectin CGL2.", PLOS PATHOGENS JAN 2010 LNKD- PUBMED:20062796, vol. 6, no. 1, E1000717, January 2010 (2010-01), pages 1-15, XP002629521, ISSN: 1553-7374
- WEI J-Z ET AL: "BACILLUS THURINGIENSIS CRYSTAL PROTEINS THAT TARGET NEMATODES", PROCEEDINGS OF THE NATIONAL ACADEMY OF SCIENCES OF THE UNITED STATES (PNAS), NATIONAL ACADEMY OF SCIENCE, US, vol. 100, no. 5, 4 March 2003 (2003-03-04) , pages 2760-2765, XP009084270, ISSN: 0027-8424, DOI: DOI:10.1073/PNAS.0538072100
- ROTH-WALTER F ET AL: "Mucosal targeting of allergen-loaded microspheres by Aleuria aurantia lectin", VACCINE, ELSEVIER LTD, GB, vol. 23, no. 21, 15 April 2005 (2005-04-15), pages 2703-2710, XP027652095, ISSN: 0264-410X [retrieved on 2005-04-15]
- ROTH-WALTER F ET AL: "Targeting antigens to murine and human M-cells with Aleuria aurantia lectin-functionalized microparticles", IMMUNOLOGY LETTERS, ELSEVIER BV, NL, vol. 100, no. 2, 15 September 2005 (2005-09-15), pages 182-188, XP027672150, ISSN: 0165-2478 [retrieved on 2005-09-15]
- BIRGIT SAUERZAPFE ET AL: "Chemo-enzymatic synthesis of poly-N-acetyllactosamine (poly-LacNAc) structures and their characterization for CGL2-galectin-mediated binding of ECM glycoproteins to biomaterial surfaces", GLYCOCONJUGATE JOURNAL, KLUWER ACADEMIC PUBLISHERS, BO, vol. 26, no. 2, 29 August 2008 (2008-08-29), pages 141-159, XP019676223, ISSN: 1573-4986

## Description

The present invention relates to the use of glycan-binding polypeptides for treating and/or preventing helminthic infections. Moreover, the present invention is directed to corresponding pharmaceutical compositions, food products and animal feed comprising glycan-binding polypeptides.

Parasitic worms or helminths are eukaryotic parasites that live inside their host and feed of living cells. They are categorized into cestodes, trematodes and nematodes. Typical diseases mediated by helminths are ascariasis, dracunculiasis, elephantiasis, hookworm, lymphatic filariasis, onchocerciasis, schistosomiasis and trichuriasis, The "roundworms" or "nematodes" are the most diverse phylum of pseudocoelomates and one of the most diverse of all animals. Nematode species are difficult to distinguish; over 80,000 have been described, of which over 15,000 are parasitic. It has been estimated that the total number of roundworm species might be more than 500,000. Nematodes are ubiquitous in freshwater, marine and terrestrial environments. Most of them are predators of bacteria and fungi. Parasitic forms include pathogens of plants, animals and also humans.

*Caenorhabditis elegans* (*C. elegans*) is a model nematode and is unsegmented, vermiform, bilaterally symmetrical, with a cuticle integument, four main epidermal cords and a fluid-filled pseudocoelomate cavity. In the wild it feeds on bacteria that develop on decaying vegetable matter. The glycobiology of the organism is intensely investigated (reviewed in Berninsone, Wormbook, 1-22, 2006; Schachter, Curr. Opin. Struct. Biol., 14:607, 2004). In particular, the N-glycosylation pattern of *C. elegans* is well characterized and was recently reviewed in Paschinger et al. (Carbohydrate Res., 343:2041, 2008). As a characteristic modification of nematode (and mollusc) N-glycans, Hannemann et al. (Glycobiology, 16:874, 2006) isolated and structurally characterized D-galactopyranosyl-β-1,4-L-fucopyranosyl-α-1,6-D-GlcNAc (Gal-Fuc) epitopes at the N-linked GlcNAc residue of the N-glycan core.

Lectin-based defence systems against predators, parasites and pathogens are widespread in nature. Lectins function in defence either as direct effectors by their toxicity towards target organisms or as opsonins by labeling target organisms for other effector molecules or cells. Former mechanism is common for plant lectins directed against herbivores whereas latter mechanism is common for animal lectins directed against pathogens. Recently some examples of animal lectins acting as direct effectors against bacteria and fungi were reported (Cash et al., Science, 313:1126, 2006; Kohatsu et al., J. Immunol., 177:4718, 2006).

Fungi contain a large number of lectins of different specificity and folds (Goldstein and Winter, Mushroom lectins, in: Comprehensive Glycoscience: From Chemistry to Systems biology, Elsevier Ltd. 2007). Many of these lectins are specifically produced in the reproductive organs, the fruiting bodies, and lack a classical signal sequence for secretion. The physiological role of these fruiting body lectins is unclear. The absence of a fruiting phenotype upon inactivation of the respective genes or mRNAs argues against an endogenous role of these lectins in development (Nowrousian et al., BMC Microbiology, 5:64, 2005; Wälti et al., Eukaryot. Cell, 5:732-744, 2006). The toxicity of a variety of these lectins against a number of organisms (insects, nematodes, amoebae) with fungal-feeding relatives suggests that fruiting body lectins may have a role in the defence of fungi against predators and parasites (Trigueros et al., BBA, 1621:292-298, 2003; Zhao et al., Environ. Toxicol. Pharmacol., 28:265-268, 2009).

Nematodes and plants share the α-1,3-fucosylation on the N-linked GlcNAc-residue of N-glycan cores. This glycoepitope is one of the main allergens of pollen (Wicklein et al., Biol. Chem. 385:397, 2004). It is speculated ("hygiene hypothesis") that the reduced exposition of 1^{st} world people to helminths including nematodes in the course of todays hygiene standards is one of the reasons for the increasing number of people with pollen and other types of allergies. Recent success in the treatment of such allergies by transient infections of patients with relatively harmless parasitic nematodes supports this hypothesis but could also have other reasons (Reddy and Fried, Parasitol. Rev., 104:217, 2009). In some parasitic helminths, the Fuc-α-1,3-GlcNAc epitope is also present on the N-glycan antenna and has been implicated in host immune suppression (van Die and Cummings, Glycobiology, 20:2, 2010).

US Patent No. 5,707,817 teaches the use of a diagnostic reagent comprising the mono-saccharide β-tyvelose joined to at least one further saccharide to form an oligosaccharide having at least one β-tyvelose terminal residue conjugated to a carrier for presenting said oligosaccharide to an antibody recognizing the terminal β-tyvclose for the diagnostic purpose of detecting *Trichinella spiralis* infections. The authors also very generally speculate on *"therapeutic agents based on the knowledge that β-tyvelose is produced in Trichinella spiralis parasites".*

US 2002/0160021 A1 and US Patent 7,063,848 B2, both by L.H. Semprevio describe protective vaccines against *Schistosoma mansoni, Fasciola hepatica,* cestoidean, protozoan pathogens and possibly pathogenic nematodes that comprise a carrier group coupled to an oligosaccharide obtained from an eukaryotic surface lipoglycan which comprises a lipid group, one or more fucose groups, three to five galactosamine groups per fucose, two to four glucosamine groups per fucose, one to two galactose groups per fucose, one to two glucose groups per fucose, one to two rhamnose groups per fucose and one to three mannose groups per fucose. A proof of concept for nematodes is absent.

It is the object of the present invention to provide new means for treating and/or preventing helminthic, in particular nematodes infections. Furthermore, it is the object of this invention to provide functional food products and animal feed for treating and/or preventing helminthic, in particular nematode infections.

It was surprisingly found that glycan-binding polypeptides can be used for treating and/or preventing helminthic, in particular nematode infections.

Therefore, in a first aspect, the above object is solved by the use of glycan-binding polypeptides according to claim 1.

The term glycan, as used herein, refers to mono-, oligo- or polysaccharides of homogenous or heterogenous composition with regard to linkage, substitution, modification or identity of the monosaccharide building blacks. In nature glycans are categorized into N-, O- or lipoglycans depending on the type of bond and conjugate component, e.g. a polypeptide or lipid.

The term glycoconjugate, as used herein, refers to enzymatically (*in vitro* or *in vivo*) or chemically produced conjugates of glycans and carrier molecules, e.g. selected from proteins, lipids, any other type of molecule and also cells. Cellular glycan conjugates are preferably presented on the cell surface. Typical embodiments of glycoconjugates are chemical conjugates of glycans to carrier proteins, e.g. inactivated bacterial toxins or keyhole limpet hemocyanin (KLH), and genetically engineered *Escherichia coli* or *Salmonella typhimurium* strains displaying specific glycans as part of their lipopolysaccharide (LPS) or lipooligosaccharide (LOS).

Hence, glycan-binding polypeptides according to the invention are any amino acid and peptide bond-based compounds that specifically bind to at least one glycan. The term encompasses oligopeptides as well as chemical derivatives of poly- and oligopeptides, e.g. oligo/polypeptides comprising one or more amino acid analogs as well as conjugates of oligo/polypeptides and non-amino acid-based chemical moieties. The scope of the term is limited by the necessity that glycan-binding should essentially be mediated by the peptide component. Typical embodiments of glycan-binding polypeptides are lectins, antibodies, fragments or functional derivatives of antibodies and antibody-like binding proteins.

In a preferred embodiment the glycan-binding polypeptides for medical use according to the invention are selected from N-glycan-binding polypeptides.

In a more preferred embodiment the N-glycan-binding polypeptides for medical use bind galactoside-containing oligo/polysaccharides and/or glycoconjugates, preferably D-galacto-pyranosyl-β-1,4-containing oligo/polysaccharides and/or glycoconjugates, preferably D-galacto-pyranosyl-β-1,4-L-fucopyranosyl-containing oligo/polysaccharides and/or glycoconjugates, more preferably D-galacto-pyranosyl-β-1,4-L-fucopyranosyl-α-1,6-GlcNAc-containing oligo/polysaccharides and/or glycoconjugates, most preferably GnGnF⁶Gal- and/or MMF⁶Gal-containing oligosaccharides and glycoconjugates. An example of such an N-glycan-binding polypeptide is lectin CGL2, which is described in more detail further below.

In a more preferred embodiment the N-glycan-binding polypeptides for medical use bind fucoside-containing oligo/polysaccharides and/or glycoconjugates, preferably L-fucopyranosyl-α-1,3-containing oligo/polysaccharides and/or glycoconjugates, more preferably L-fucopyranosyl-α-1,3-GlcNAc-containing oligo/polysaccharides and/or glycoconjugates, most preferably GnGnF³- and/or MMF³-containing oligosaccharides and glycoconjugates. An example of such an N-glycan-binding polypeptide is lectin RedA, which is described in more detail further below.

In an additional preferred embodiment the glycan-binding polypeptides for medical use are selected from O-glycan-binding polypeptides.

In a more preferred embodiment the O-glycan-binding polypeptides for medical use bind galactoside-containing oligo/polysaccharides and/or glycoconjugates, preferably D-galacto-pyranosyl-β-1,3-containing oligo/polysaccharides and/or glycoconjugates, more preferably D-galacto-pyranosyl-β-1,3-N-acetyl-D-galactosamino-pyranosyl-containing oligo/polysaccharides and/or glycoconjugates, most preferably D-galacto-pyranosyl-β-1,3-N-acetyl-D-galactosamino-pyranosyl-O-Ser/Thr compounds. Examples of such O-glycan-binding polypeptides are lectins XCL and TAP1, both of which are described in more detail further below.

In an additional preferred embodiment the glycan-binding polypeptides for medical use are selected from lipoglycan-binding polypeptides.

In a more preferred embodiment the lipoglycan-binding polypeptides for medical use bind galactoside-containing oligo/polysaccharides and/or glycoconjugates, preferably D-galacto-pyranosyl-α-1,3-containing oligo/polysaccharides and/or glycoconjugates, more preferably D-galacto-pyranosyl-α-1,3-N-acetyl-D-galactosamino-pyranosyl-containing oligo/polysaccharides and/or glycoconjugates, most preferably D-galacto-pyranosyl-α-1,3-N-acetyl-D-galactosamino-pyranosyl-β-1,4-N-acetyl-D-glucosamino-pyranosyl-containing oligo/polysaccharides and/or glycoconjugates. An example of such a lipoglycan-binding polypeptide is MOA agglutinin, which is described in more detail further below.

It was surprisingly found and experimentally demonstrated that helminthic, in particular nematode glycans are particularly well suited as targets for glycan-binding polypeptides having medical utility for treating and/or preventing helminthic infections. Therefore, in a preferred embodiment the glycan-binding polypeptides for medical use according to the invention are characterized in that they bind to at least one nematode glycan, preferably produced by nematodes selected from the family Trichostrongylidae, preferably *Haemonchus contortus, Trichostrongylus colubriformis*, *Teladorsagia circumcincta, Cooperia oncophora, Nematodirus battus, Ostertagia leptospicularis, Chabertia ovina*, *Oesophagostomum dentatum,* and nematode species *Trichinella spiralis, Trichuris trichuria, Angiostrongylus vasorum, Ancylostoma caninum, Ancylostoma duodenale, Ancylostoma ceylanicum, Necetor americanus, Dictyocaulus* spp., *Ascaris lumbricoides, Ascaris suum, Wuchereria bancrofti, Brugia malayi, Loa Ion, Enterobius vermicularis, Dirofilaria immitis, Onchocerca volvulus and Strongyloides stercoralis.*

In the context of the above definition of glycan-binding polypeptides, the polypeptides for medical use are preferably selected from the group consisting of lectins, antibodies, fragments or functional derivatives of lectins and antibodies and antibody-like binding proteins.

The term "lectin" as used herein encompasses any amino acid and peptide bond-based compound having specific binding affinity to carbohydrates. Typically it relates to non-antibody polypeptides found in nature featuring specific carbohydrate binding. The term "lectin" includes functional fragments and derivatives thereof, the latter terms being defined in analogy to the same terms used in the context of antibodies below.

Furthermore, in one aspect the present invention relates to antibodies, functional fragments and functional derivatives thereof that specifically bind a glycan for medical use as defined above. These are routinely available by hybridoma technology (Kohler and Milstein, Nature 256, 495-497,1975), antibody phage display (Winter et al., Annu. Rev. Immunol. 12, 433-455, 1994), ribosome display (Schaffitzel et al., J. Immunol. Methods, 231,119-135, 1999) and iterative colony filter screening (Giovannoni et al., Nucleic Acids Res. 29, E27, 2001) once the target glycan antigen is available. Typical proteases for fragmenting antibodies into functional products are well-known. Other fragmentation techniques can be used as well as long as the resulting fragment has a specific high affinity and, preferably a dissociation constant in the micromolar to picomolar range. Examples of glycan-binding antibodies are the 'lgG fraction of anti-Peroxidase' rabbit antiserum (Cat. No. 200-4138, Rockland Inc., USA) and Anti-Peroxidase antibody produced in rabbit' (Cat. No. P7899, Sigma-Aldrich Co., USA).

A very convenient antibody fragment for glycan-binding applications is the single-chain Fv fragment, in which a variable heavy and a variable light domain are joined together by a polypeptide linker. Other antibody fragments for binding to glycans according to the present invention include Fab fragments, Fab₂ fragments, miniantibodies (also called small immune proteins), tandem scFv-scFv fusions as well as scFv fusions with suitable domains (e.g. with the Fc portion of an immunoglobulin). For a review on certain antibody formats, see Holliger P, Hudson PJ. (Nat. Biotechnol., 23:1126-36, 2005).

The term "functional derivative" of an antibody for use in the present invention is meant to include any antibody or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative has substantially the same binding affinity as to its original antigen and, preferably, has a dissociation constant in the micro-, nano- or picomolar range.

In a preferred embodiment, the antibody, fragment or functional derivative thereof for use in the invention is one that is selected from the group consisting of polyclonal antibodies, monoclonal antibodies, chimeric antibodies, humanized antibodies, CDR-grafted antibodies, Fv-fragments, Fab-fragments and Fab₂-fragments.

For a review of antibody-like binding proteins see Binz et al. on engineering binding proteins from non-immunoglobulin domains in Nature Biotechnology, 23:1257-1268, 2005. The term "aptamer" describes nucleic acids that bind to a polypeptide with high affinity. Aptamers can be isolated from a large pool of different single-stranded RNA molecules by selection methods such as SELEX (see, e.g., Jayasena, Clin. Chem., 45:1628 - 1650, 1999; Klug and Famulok, M. Mol. Biol. Rep., 20:97 - 107, 1994; US 5,582,981). Aptamers can also be synthesized and selected in their mirror form, for example, as the L-ribonucleotide (Nolte et al., Nat. Biotechnol., 14:1116 - 1119, 1996; Klussmann et al., Nat. Biotechnol., 14:1112 - 1115, 1996). Forms isolated in this way have the advantage that they are not degraded by naturally occurring ribonucleases and, therefore, have a greater stability.

Another antibody-like binding protein and alternative to classical antibodies are the so-called "protein scaffolds", for example, anticalines, that are based on lipocaline (Beste et al., Proc. Natl. Acad. Sci. USA, 96:1888 - 1903, 1999). The natural ligand binding sites of lipocalines, for example, of the retinol-binding protein or bilin-binding protein, can be changed, for example, by employing a "combinatorial protein design" approach, and in such a way that they bind selected haptens (Skerra, Biochem. Biophys. Acta, 1482:337 - 350, 2000). For other protein scaffolds it is also known that they are alternatives for antibodies (Skerra, J. Mol. Recognit, 13:167 - 287, 2000). (Hey, Trends in Biotechnology, 23:514-522, 2005; EP 1 892 248 A1/"fynomers").

In summary, the term antibody-like binding proteins is meant to include the above protein-derived alternatives for antibodies, e.g. affilines, anticalines, aptamers and fynomers, that specifically recognize a target, preferably a glycan for medical use.

Described is also a hybridoma cell line, expressing a monoclonal antibody binding to a glycan as defined above for medical use.

It has been surprisingly found that polyvalent glycan-binding polypeptides are of advantage, e.g. more toxic to helminths, over corresponding univalent polypeptides when used as active component in medicaments for treating and/or preventing helminthic, preferably nematode and immune diseases. Without wishing to be bound by theory, it is presently assumed that polyvalent binding results in crosslinking of surface-exposed glycans which triggers uptake of the glycan-binding polypeptide by endocytosis and/or leads to the activation of a signal cascade. A dependency of lectin-mediated cellular toxicity on polyvalency of a lectin has been demonstrated (Yang et al., J. Mol. Biol., 387:694-705, 2009). Binding sites on polyvalent glycan-binding polypeptides can be directed towards the same glycan or towards different glycans. An example of latter type of polyvalent glycan-binding polypeptide is XCL which is described in more detail further below.

Because of the above, a preferred embodiment of the present invention is directed to the medical use of polyvalent glycan-binding polypeptides as defined above, preferably having binding moieties for binding at least two or more, more preferably three or more, most preferably four or more glycans.

The claimed medical utility has been demonstrated for a number of lectins, in particular fungal lectins. In a most preferred embodiment, the present invention is directed to the medical use of lectins, preferably selected from the group of fungal lectins, preferably lectins from *Coprinopsis cinerea, Xerocomus chrysenteron, Marasmius oreades, Aleuria aurantia* or *Sordaria macrospora,* more preferably lectins selected from CGL1, CGL2, RedA (CCL2), CCL1, XCL, MOA, AAL and TAP1.

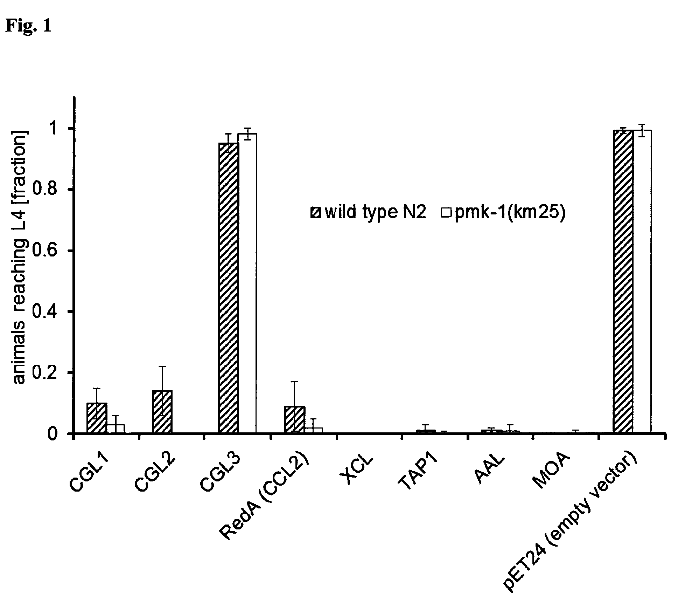

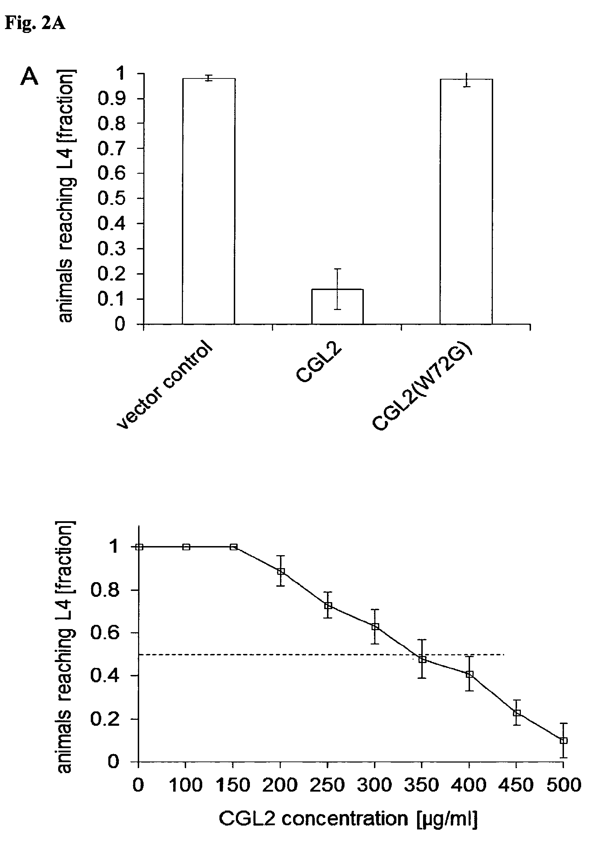

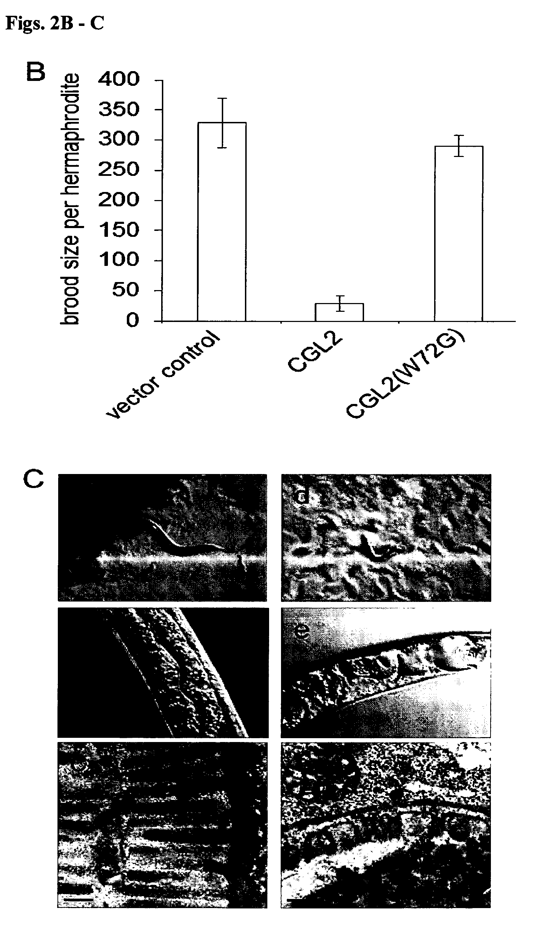

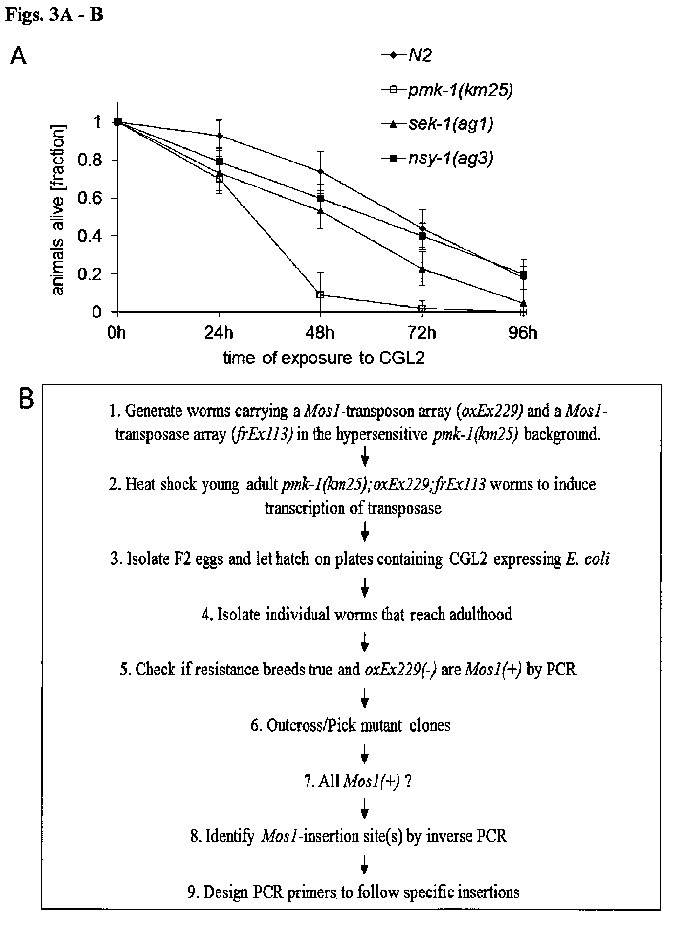

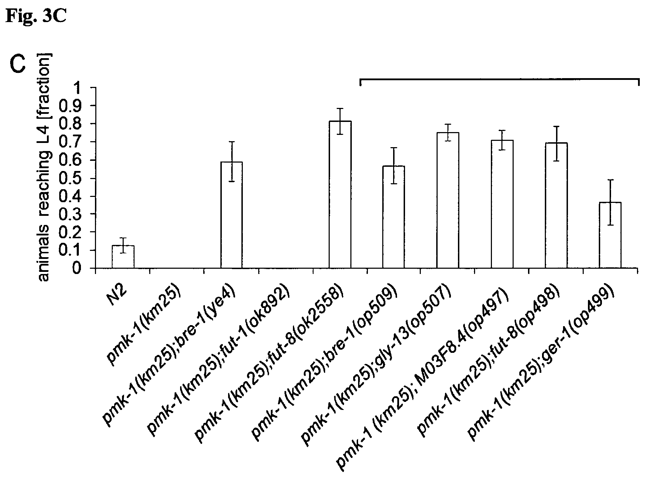

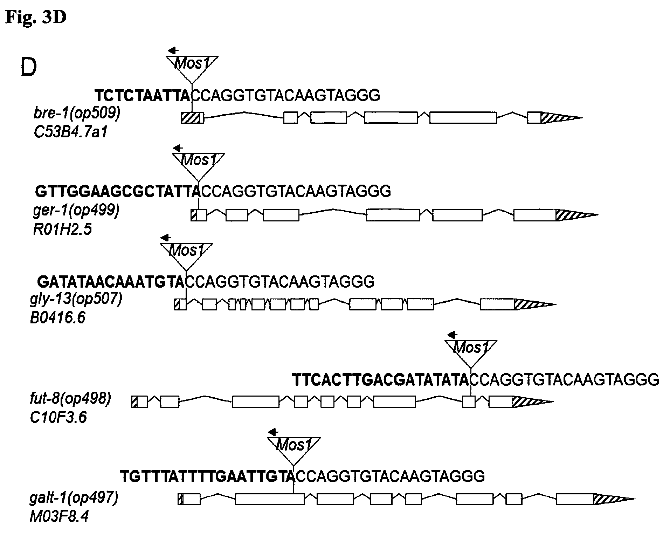

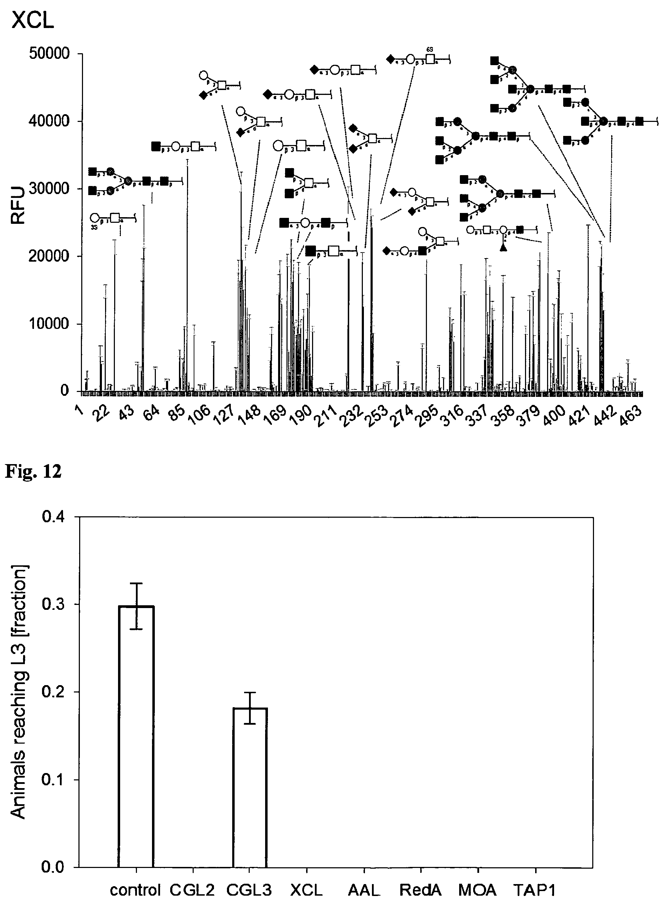

Galectins CGL1 and CGL2 (Genbank AAF34731 and AAF34732) from *C. cinerea* are β-galactoside binding lectins with a conserved carbohydrate binding domain (Leffler et al., Glycoconj. J., 19:433, 2004). Animal galectins probably have a dual role in innate immunity of animals by recognition of damage- and pathogen-associated molecular patterns (Sato et al., Immunol Rev., 230:172, 2009). Fungal galectins were first reported for *Coprinopsis cinerea* (*C. cinerea*), an inky cap mushroom which is, together with *Schizophyllum commune,* one of two homobasidiomycete fungi that are commonly used in research as models for this group of organisms. The *C. cinerea* genome, that has recently been sequenced, codes for two isogalectins, CGL1 and CGL2 and a galectin-related protein CGL3 (Genbank ABD64675). These proteins are induced to high levels during fruiting body formation (Boulianne et al., Microbiology, 1841-1853, 2000). Co-silencing of the mRNAs for CGL1 and CGL2 did not result in an obvious defect in fruiting body formation (Wälti et al., Eukaryot Cell, 5:732-744, 2006). Both CGL1 and CGL2, but not CGL3, demonstrate high toxicity towards *C. elegans* in toxicity assays using lectin-expressing *E. coli* cells as sole food source (see Examples below, Fig. 1 and 2). Using a forward genetic screen and testing of available *C. elegans* glycosylation mutants, the target glycan recognized by CGL2 in *C. elegans* was identified as Gal-β-1,4-Fuc-α-1,6 on the Asn-linked GlcNAc-residue of N-glycan cores (Fig. 3 to 7, Table 1 below). CGL2, but not CGL3, is also highly toxic to the bacterivorous stages of the parasitic nematode *Haemonchus contortus* (see Examples below, Fig. 12).

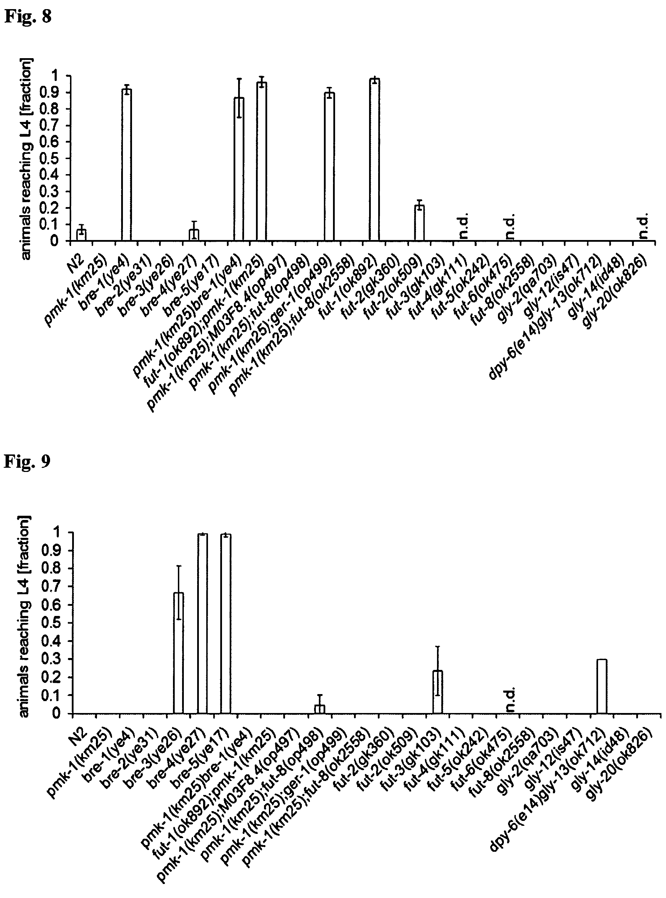

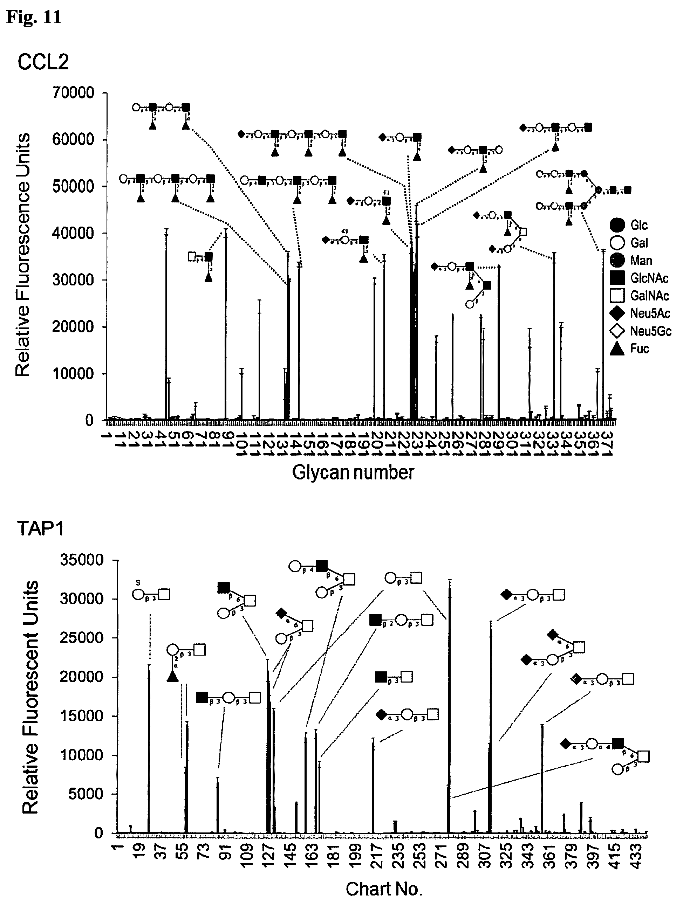

Lectin RedA (CCL2) (Genbank ACD88750) was isolated from *C. cinerea* fruiting body extracts by affinity chromatography using immobilized horseradish peroxidase (HRP), a plant glycoprotein (Wälti et al., unpublished). Subsequent analysis showed that it is a cytoplasmic protein highly induced during fruiting body formation and binding specifically to Fuc-α-1,3-GlcNAc on plant N-glycan cores (see Fig. 11). RedA (CCL2) is toxic towards *C. elegans* (see Fig. 1) and *H. contortus* (see Fig. 12). Testing of available C. *elegans* glycosylation mutants revealed that nematotoxicity is dependent on the Fuc-α-1,3 on the N-linked GlcNAc-residue of N-glycan cores (see Fig. 8, Table 1). The isolectin CCL1 (Genbank HQ267703) from the same organism was also analyzed and showed essentially the same properties as RedA with regard to glycan-binding and toxicity (data not shown).

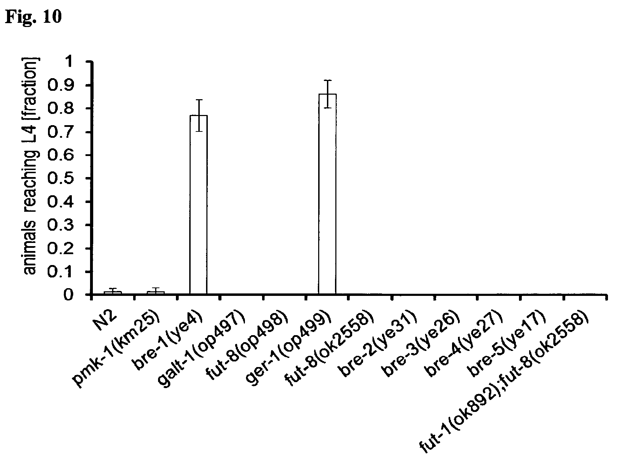

Lectin AAL (Genbank BAA00451) is a β-propeller lectin from the ascomycete *Aleuria aurantia.* The protein is a homodimer containing five binding sites for terminal fucose in either α-1,2- or α-1,3-linkage on each subunit (Wimmerova et al, J. Biol. Chem., 278:-27059, 2003). Orthologs from other ascomycetes may differ slightly in their preference for the linkage of the terminal fucose residue (Matsumura et al, Anal. Biochem., 386:217, 2009). Feeding of AAL-expressing *E*. *coli* is toxic to *C. elegans* (Fig.1) and *H. contortus* (see Fig. 12). Testing of available *C. elegans* glycosylation mutants revealed that the nematotoxicity is dependent on GDP-fucose biosynthesis (Fig. 10, Table 1).

Agglutinin MOA (Genbank AAL47680) is a homodimeric lectin from the homobasidiomycete *Marasmius oreades* with a putative catalytic domain which is at the same time the dimerization domain (Grahn et al., J. Mol. Biol., 390:457, 2009). The lectin domain adopts a RicinB-fold and contains three binding sites for the xenotransplantation epitope Gal-α-1,3-Gal. This epitope is found on some species of C. *elegans* glycosphingolipids (species D in Griffitts et al, Science, 307:922, 2005). The lectin has homologs in other fungi with slightly different carbohydrate binding specificities of the lectin domain (Tateno et al, Biochem. J., 382:667, 2004). Feeding of MOA-expressing *E. coli* is toxic to *C. elegans* (see Fig.1) and *H. contortus* (see Fig. 12). Testing of *C. elegans* glycosylation mutants revealed that the nematotoxicity is dependent on the biosynthesis of a specific glycosphingolipid species that is different from the one recognized by the nematotoxic *Bacillus thuringiensis* crystal toxin CRY5B (Griffitts et al, Science, 307:922, 2005; see Fig. 9, Table 1). Gal-α-1,3-GalNAc-specific antibodies have recently been implicated in the protection of sheep from *H. contortus* (van Stijn et al, Int. J. Parasitol., 40:215, 2010).

Lectin XCL (Genbank AAL73235) form *Xerocomus chrysenteron* and lectin TAP1 (Genbank CAH03681) from *Sordaria macrospora* are dual specificity lectins with orthologs in many fungi and also in lower plants (Peumans et al., Plant Physiol., 144:637, 2007). XCL is a homotetramer and possibly has one binding site for Gal-β-1,3-GalNAc/GalNAc and another one for GlcNAc on each subunit (Birck et al., J. Mol. Biol., 344:1409, 2004; Leonidas et al., J. Mol. Biol., 368:1145, 2007). Former glycoepitope is found on O-glycans of *C. elegans* and also of mammals. XCL has insecticidal activity (Trigueros et al., BBA, 1621:292, 2003). Both XCL and TAP1 reveal strong nematotoxicity when fed to *C. elegans* (see Fig. 1) and *H. contortus* (see Fig. 12).

The glycan-binding polypeptides of the invention, e.g. lectins (see Figs. 1 and 12) and glycan-binding antibodies (see Fig. 13), have demonstrated toxicity to helminths, in particular nematodes in toxicity assays.

Recent success in the treatment of allergies by transient infections of patients with relatively harmless parasitic nematodes demonstrated the utility of nematode components for treating and/or preventing immune diseases (Reddy and Fried, Parasitol. Rev., 104:217,2009). It is the glycans of helminths, preferably nematodes that are the components responsible for the positive immune modulation.

More preferably, said helminthic, preferably nematode infection is an infection resulting from a helminth selected from the family Trichostrongylidae, preferably *Haemonchus contortus, Trichostrongylus colubriformis, Teladorsagia circumcincta, Cooperia oncophora, Nematodirus battus, Ostertagia leptospicularis, Chabertia ovina, Oesophagostomum dentatum,* and nematode species *Trichinella spiralis, Trichuris trichuria, Angiostrongylus vasorum, Ancylostoma caninum, Ancylostoma duodenale, Ancylostoma ceylanicum, Necator americanus, Dictyocaulus* spp., *Ascaris lumbricoides, Ascaris suum, Wuchereria bancrofti, Brugia malayi, Loa loa, Enterobius vermicularis, Dirofilaria immitis, Onchocerca volvulus and Strongyloides stercoralis.*

As mentioned above, in particular antibodies may function as glycan-binding polypeptides having the medical utility claimed. These may be generated *in vivo* in the vertebrate to be treated.

In a further aspect, the present invention relates to a pharmaceutical composition comprising at least one glycan-binding polypeptide, preferably one that binds N-glycans, preferably N-glycans selected from the group consisting of galactoside-containing oligo/polysaccharides and/or glycoconjugates, preferably galacto-pyranosyl-β-1,4-containing oligo/polysaccharides and/or glycoconjugates, preferably D-galacto-pyranosyl-β-1,4-L-fucopyranosyl-containing oligo/polysaccharides and/or glycoconjugates, more preferably D-galacto-pyranosyl-β-1,4-L-fucopyranosyl-α-1,6-GlcNAc-containing oligo/polysaccharides and/or glycoconjugates, most preferably GnGnF⁶Gal- and/or MMF⁶Gal-containing oligosaccharides and glycoconjugates.

In a further preferred embodiment, the present invention relates to a pharmaceutical composition comprising at least one N-glycan-binding polypeptide, preferably one that binds fucoside-containing oligo/polysaccharides and/or glycoconjugates, preferably L-fucopyranosyl-α-1,3-containing oligo/polysaccharides and/or glycoconjugates, more preferably L-fucopyranosyl-α-1,3-GlcNAc-containing oligo/polysaccharides and/or glycoconjugates, most preferably GnGnF³-and/or MMF³-containing oligosaccharides and glycoconjugates.

In a preferred embodiment, the present invention relates to a pharmaceutical composition comprising at least one O-glycan-binding polypeptide, preferably one that binds galactoside-containing oligo/polysaccharides and/or glycoconjugates, preferably D-galacto-pyranosyl-β-1,3-containing oligo/polysaccharides and/or glycoconjugates, more preferably D-galacto-pyranosyl-β-1,3-N-acetyl-D-galactosomino-pyranosyl-containing oligo/polysaccharides and/or glycoconjugates, most preferably D-galacto-pyranosyl-β-1,3-N-acetyl-D-galactosamino-pyranosyl-O-Ser/Thr compounds.

In a another preferred embodiment, the present invention relates to a pharmaceutical composition comprising at least one lipoglycan, preferably selected from galactoside-containing oligo/polysaccharides and/or glycoconjugates, preferably D-galacto-pyranosyl-α-1,3-containing oligo/polysaccharides and/or glycoconjugates, more preferably D-galacto-pyranosyl-α-1-3-N-acetyl-D-galactosamino-pyranosyl-containing oligo/polysaccharides and/or glycoconjugates, most preferably D-galacto-pyranosyl-α-1,3-N-acetyl-D-galactosamino-pyranosyl-β-1,4-N-acetyl-D-glucosamino-pyranosyl-containing oligo/polysaccharides and/or glycoconjugates.

Furthermore, it is preferred that the glycan-binding polypeptide binds to at least one nematode glycan produced by nematodes selected from the family Trichostrongylidae, preferably *Haemonchus contortus, Trichostrongylus colubriformis, Teladorsagia circumcincta, Cooperia oncophora, Nematodirus battus, Ostertagia leptospicularis Chabertia ovina, Oesophagostomum dentatum,* and nematode species *Trichinella spiralis, Trichuris trichuria, Angiostrongylus vasorum, Ancylostome caninum, Ancylostome duodenale, Ancylostoma ceylanicum, Necator americanus, Dictyocaulus* spp., *Ascaris lumbricoides, Ascaria suum, Wuchereria bancrofti, Brugia malayi, Loa loa*, *Enterobius vermicularis, Dirofilaria immitis, Onchocerca volvulus and Strongyloides stercoralis.*

More preferably, the glycan-binding polypeptides of the pharmaceutical composition are selected from the group consisting of lectins, antibodies, fragments or functional derivatives of antibodies and antibody-like binding proteins.

More preferably, the glycan-binding polypeptides of the pharmaceutical composition are polyvalent and thus bind to at least two or more, preferably three or more, more preferably four or more glycans.

Most preferably, the glycan-binding polypeptides of the pharmaceutical composition are lectins, preferably selected from the group of fungal lectins, preferably lectins from *Coprinopsis cinerea, Xerocomus chrysenteron, Marasmius oreades, Aleuria aurantia* or *Sordaria macrospora,* more preferably lectin CGL1, CGL2, RedA (CCL2), CCL1, XCL, MOA, AAL or TAP1.

For the pharmaceutical compositions it is preferred that the glycan-binding polypepides are displayed on the surface of bacterial cells, preferably enterobacteria, more preferably human enterobacterial cells, most preferably *Escherichia coli* or *Salmonella typhimurium.*

A further aspect of the invention relates to a food product or animal feed comprising isolated glycan-binding polypeptides, preferably N-, O- and/or lipoglycan-binding polypeptides, more preferably those glycan-binding polypeptides described above for providing the treatment and/or preventive medical effects described above, in particular anti-helminthic effects. Preferably, the invention relates to a food or feed for humans or animals, preferably livestock, comprising isolated glycan-binding polypeptides as defined above and a physiologically acceptable excipient and/or food stuff, preferably enterobacteria, more preferably human or livestock enterobacterial cells, most preferably *Escherichia coli* or *Salmonella typhimurium.* For example, such a food or feed would greatly reduce helminth colonisation in humans or livestock, respectively, and/or stimulate or suppress the immune system against helminth-related antigens.

In a further aspect, the present invention relates to methods for identifying helminths susceptible to toxicity mediated by glycan-binding polypeptides.

The term "specific binding" as used throughout the specification and claims is meant to exclude compounds that will unspecifically bind to many or most carbohydrate and/or non-carbohydrate compounds.

The present disclosure also relates to a method for treating and/or preventing helminthic, preferably nematode infections and/or immune diseases, comprising administration of a glycan-binding polypeptide, pharmaceutical composition, food or feed of the present invention to a human or animal in need thereof in a physiologically active amount.

For therapeutic and/or prophylactic use the pharmaceutical compositions of the invention may be administered in any conventional dosage form in any conventional manner. Routes of administration include, but are not limited to, intravenously, intramuscularly, subcutaneously, intranasally, intrasynovially, by infusion, sublingually, transdermally, orally (e.g. tablet, gavage), topically or by inhalation. The preferred modes of administration are oral, intravenous and intranasal, oral and intranasal being most preferred.

The glycan-binding polypeptides of the invention may be administered alone or in combination with adjuvants that enhance stability and/or immunogenicity of the medically effective compounds, facilitate administration of pharmaceutical compositions containing them, provide increased dissolution or dispersion, increase propagative activity - if cells are involved, e.g. cells producing the medically effective compounds, provide adjunct therapy, and the like, including other active ingredients.

In the past, immunizations of animals with recombinant digestive proteases of parasitic helminths have been partially successful in terms of reducing the egg count in the feces and the worm burden in the animals (see e.g. Reszka et al, Exp. Parasitol., 117:208, 2007; Loukas et al, PLoS Medicine, 2:e295, 2005). However, these trials did not reach the degree of protection achieved by immunization with some native gut proteases (reviewed in Pearson et al, Biol. Chem., 391:901, 2010). It is hypothesized that part of this difference is due to the lack of nematode-specific glycosylation in the recombinant proteins.

Recombinant or native digestive proteases of parasitic helminths, preferably gut proteases of animal parasitic nematodes, more preferably aminopeptidase H11 of *Haemonchus contortus* or aspartic protease APR-1 of *Ancylostoma caninum,* may be combined with the glycans or glycan-binding polypeptides mentioned above or identified by methods of this invention. Preferably the digestive proteases, more preferably recombinant digestive proteases of parasitic helminths are combined with N-glycans, more preferably with MMF⁶Gal or MMF³, for example by using a heterologous expression system, preferably insect cells overexpressing nematode glycosyltransferases, more preferably in SF9 cells overexpressing GALT-1 or FUT-1 from *C. elegans* (the functional expression of GALT-1 in SF9 cells was demonstrated previously: PCT 50086). Such a composition combining said digestive proteases and glycan epitopes and/or glycan-binding polypeptides will provide an effective medicament, preferably a vaccine, in particular against parasitic helminths.

Therefore, the present disclosure also relates (i) to compositions, preferably pharmaceutical compositions, food products and/or animal feed comprising glycan binding polypeptides and/or glycans together with recombinant or native digestive proteases, prefereably recombinant digestive proteases, of parasitic helminths including functional fragments and functional derivatives thereof, i.e. fragments and derivatives still comprising at least some of the original protease activity, as well as directed (ii) to corresponding uses of these compositions, products and feed, preferably for treating and/or preventing helminthic infections.

Some commercially available glycoproteins naturally display some of the glycans that have been identified by this invention as being involved in glycan-mediated nematotoxicity. At the same time these glycoproteins are known to be highly immunogenic. These proteins include keyhole limpet hemocyanin (KLH) whose N-glycans carry the Gal-β-1,4-Fuc-α-1,6 epitope on the core (Wuhrer et al, Biochem. J., 378:625, 2004) and the Fuc-α-1,3-GlcNAc epitope on the antenna (Geyer et al, J. Biol. Chem., 280:40731, 2005), as well as horseradish peroxidase (HRP) whose N-glycans carry the Fuc-α-1,3-GlcNAc epitope on the core (Wuhrer et al, BBA, 1723:229, 2005). Further commercially available glycoproteins which also naturally display some of the glycans that have been identified by this invention as being involved in glycan-mediated nematotoxicity are Bromelain, Jack Bean mannosidase, Ulex europaeus agglutinin (UEA), honeybee phospholipase A2 as well as haemocyanines of mollusks such as Limulus polyphemus. All of the above comprise at least N-glycans having utility for the different aspects and embodiments of the present invention. In particular, these glycoproteins have medical use for immunization against parasitic helminths, more preferably for immunization of livestock against parasitic nematodes, most preferably for immunization of sheep against *Haemonchus contortus.*

Therefore, the present disclosure also relates to (i) the use of KLH, HRP, Bromelain, Jack Bean mannosidase, Ulex europaeus agglutinin (UEA), honeybee phospholipase A2 and haemocyanines of mollusks such as Limulus polyphemus, functional fragments or functional derivatives of these, i.e. fragments and derivatives still comprising at least one of the above-identified glycoepitopes, for preparing a medicament, preferably for treating and/or preventing helminthic infections, preferably parasitic helminthic infection as well as (ii) corresponding compositions, preferably pharmaceutical compositions, food products and/or animal feed comprising these.

Pharmaceutical dosage forms of the glycan-binding polypeptides described herein include pharmaceutically acceptable carriers and/or adjuvants known to those of ordinary skill in the art. These carriers and adjuvants include, for example, ion exchangers, alumina, aluminium stearate, lecithin, serum proteins, buffer substances, water, salts, electrolytes, cellulose-based substances, gelatine, water, pretrolatum, animal or vegetable oil, mineral or synthetic oil, saline, dextrose or other saccharide and glycol compounds such as ethylene glycol, propylene glycol or polyethylene glycol, antioxidants, lactate, etc. Preferred dosage forms include tablets, capsules, solutions, suspendsions, emulsions, reconstitutable powders and transdermal patches. Methods for preparing dosage forms are well known, see, for example, H. C. Ansel and N. G. Popovish, Pharmaceutical Dosage Forms and Drug Delivery Systems, 5th ed., Lea and Febiger (1990) and, in particular, Pastoret et al., Veterinary Vaccinology, Elsevier March 1999). Dosage levels and requirements are well-recognized in the art and may be selected by those of ordinary skill in the art from available methods and techniques suitable for a particular patient. As the skilled artisan will appreciate, lower or higher doses may be required depending on particular factors. For instance, specific doses and treatment regimens will depend on factors such as the patient's (human or animal) general health profile, the severity and course of the patient's disorder or disposition thereto, and the judgment of the treating physician or veterinarian.

In the following the invention will be demonstrated with reference to specific figures and experimental embodiments, none of which are to be considered limiting to the scope of the invention as indicated in the appended claims.

### Figures

- **Fig. 1**: is a bar chart showing the toxicity of various fungal lectins towards *C. elegans. C. elegans* wildtype (N2) and *pmk-1* mutant worms were analysed for development from L1 to L4 on lectin-expressing bacteria as described in the Experimental Procedures. The γ-coordinate is the fraction of worms reaching L4 in the presence of bacteria expressing the lectins on the X-coordinate. Fungal lectins analyzed included *C. cinerea* galectins CGL1 (Genbank AAF34731) and CGL2 (Genbank AAF34732), galectin-related lectin CGL3 (Genbank ABD64675) and lectin RedA (CCL2) (Genbank ACD88750), *Xerocomus chrysenteron* lectin XCL (Genbank AAL73235) and its ortholog TAP1 from *Sordaria macrospora* (Genbank CAH03681), *Aleuria aurantia* lectin AAL (Genbank BAA00451) and *Marasmius* oreades agglutinin MOA (Genbank AAL47680).
- **Figs. 2A** - **2C(a-f)**: shows the dose- and carbohydrate-binding dependent toxicity of C. *cinerea* galectin CGL2 towards *C. elegans.* In all of the experiments, *E. coli* BL21(DE3) cells expressing either wild type CGL2 or the carbohydrate-binding defective variant CGL2(W72G), or control transformants were fed to *C. elegans* wild type N2.
A. shows the inhibition of *C. elegans* development by CGL2. *C. elegans* L1 larvae were seeded onto lawns of above bacteria (left panel) or fed with increasing concentrations of purified CGL2 together with equal amounts of empty vector-containing BL21(DE3) in liquid culture (right panel) and scored for the fraction developing to the L4 stage within 72 h and 96 h, respectively. Columns represent the average of 6 and 12 independent experiments, respectively. Error bars indicate standard deviations.
B. shows the inhibition of reproduction of *C. elegans* by lectin CGL2. *C. elegans* adult hermaphrodites were fed with above bacteria and scored for total progeny counts per hermaphrodite. The broods of eight to ten hermaphrodites were averaged per data point. The standard deviations are indicated. None of the progeny on wild type CGL2 developed to adulthood within 96 h post hatch.
C. shows that CGL2 damages the intestine of *C*. *elegans. C*. *elegans* L4 larvae were fed with CGL2-expressing (panels d-f) and control *E. coli* BL21(DE3) cells (panels a-c) and examined after 24 h under the stereomicroscope (panels a, d) by differential interference contrast (DIC) microscopy (panels b, e) and by transmission electron microscopy (TEM) (panels c, f). The size bars in panels c and f are 200 nm.
- **Figs. 3A & B**: illustrate the results of the forward genetic screen for CGL2-resistant *C*. *elegans* mutants and a flowchart of the procedure used.
**A.** is a graph of the CGL2-sensitivity test of *pmk-1, sek-1* and *nsy-1* mutant worms defective in the p38 MAPK pathway. 10 L4 staged worms of the indicated genotypes were seeded onto a lawn of CGL2-expressing *E. coli* BL21(DE3) cells. The plates were checked for surviving animals at the indicated time points. The data points represent the average of ten independent experiments. Error bars indicate standard deviations.
**B**. describes the workflow of *Mos1* insertional mutagenesis for generating worms that carry the *Mos1* transposon array *oxEx229* and the *Mos1* transposase array *frEx113* in the CGL2-hypersensitive *pmk-1(km25)* background.
**C**. illustrates the resistance of isolated and constructed *C*. *elegans* mutants towards CGL2-mediated toxicity. *C*. *elegans* mutants of the indicated genotypes were analysed for development from L1 to L4 as outlined above. The gene appendix *(op)* and the bracket above the histogram indicate mutants isolated in the *Mos1*-screen. The other mutants were constructed by crossing.
**D.** shows the insertion sites of *Mos1* elements in CGL2-resistant mutants. Arrows above *Mos1* elements indicate the orientation of the *Mos1* primer *oJL115* used for sequencing iPCR products of mutant lysates. Bold letters indicate *C*. *elegans* genomic sequences and are followed by *Mos1* sequence. Gene models are taken from WormBase Release WS202. *bre-1(op509)* mutants have a *Mos1* insertion in the 5'-UTR of *C53B4.7α1* (SEQ ID NO: 1), located 184 bp upstream of the translational start codon. *ger-1(op499)* mutants have a *Mos1* insertion in the first exon of *R01H2.5* (SEQ ID NO: 2), located 50 bp downstream of the translational start codon. *gly-13(op507)* mutants have a *Mos1* insertion in a conserved splicing donor site flanking the first exon of *B0416.6* (SEQ ID NO: 3)*. fut-8(op498)* mutants have a *Mos1* insertion in the eighth exon of *C10F3*.*6* (SEQ ID NO: 4). *M03F8.4(op497)* mutants have a *Mos1* insertion in the second exon of *M03F8.4* (SEQ ID NO: 5).

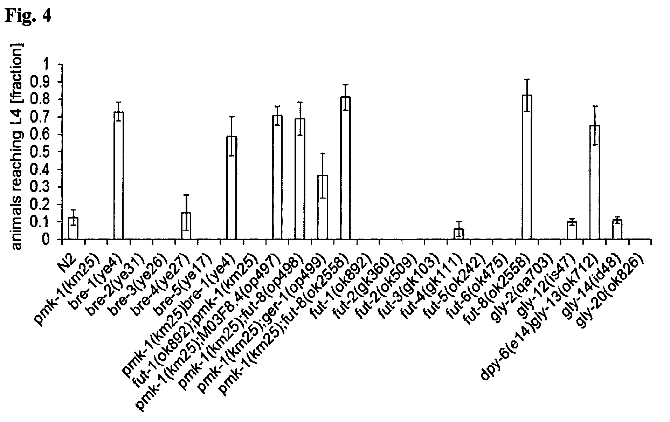

- **Fig. 4**: is a graph showing the CGL2-sensitivity of various C. *elegans* glycosylation mutants. *C. elegans* mutants of the genotypes indicated in the X-coordinate were analysed for development from L1 to L4 on CGL2-expressing *E. coli* as described in the experimental procedures below. See Table 1 below for glycosylation processes encoded by the various *C. elegans* genes.

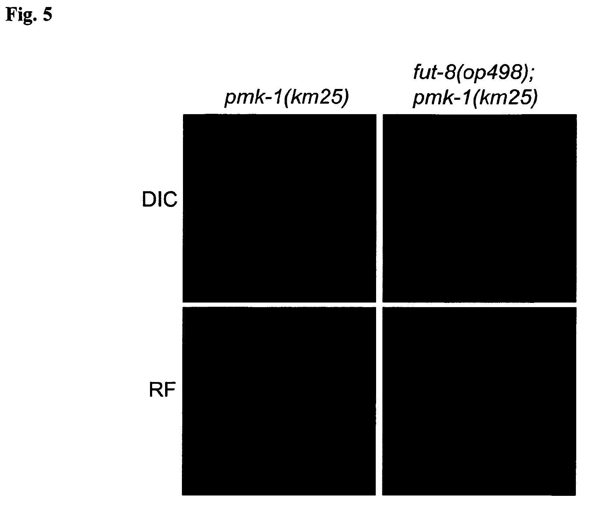

- **Fig. 5**: relates to four photographs showing the *in situ* localization of the glycoepitope recognized by CGL2 in *C*. *elegans. C*. *elegans* CGL2-sensitive *pmk-1(km25)* and CGL2-resistant *pmk-1(km25);fut-8(op498)* worms were fed with TAMRA-labeled CGL2 and examined by differential interference contrast (DIC) and red fluorescence (RF) microscopy,

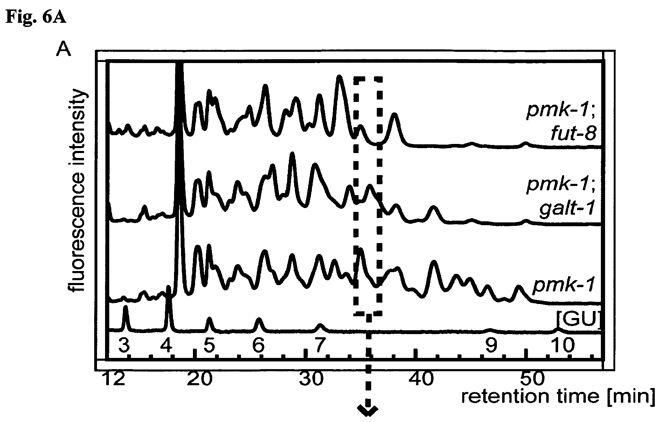

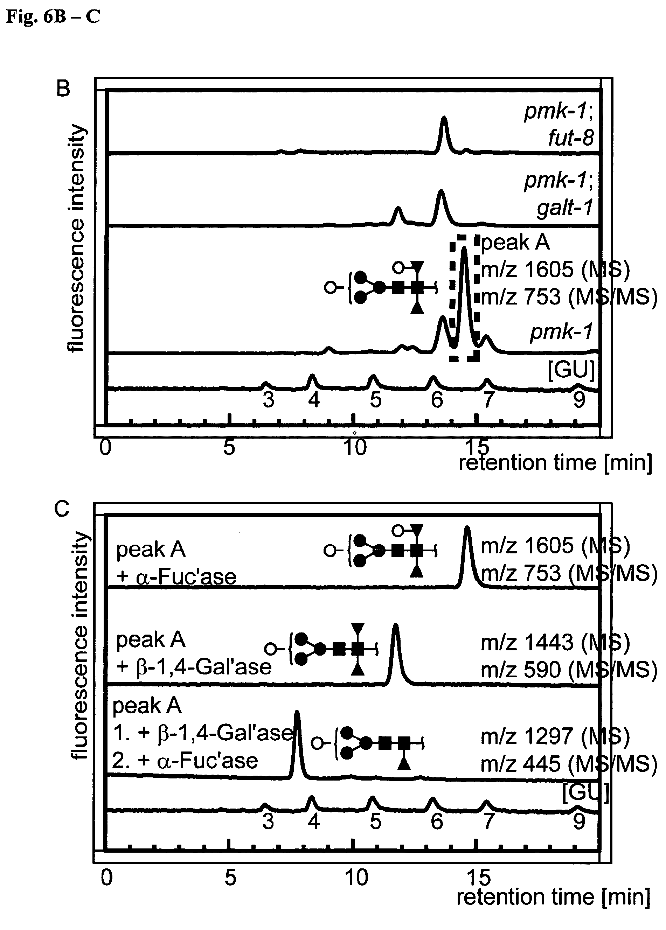

- **Figs. 6 A-C**: show a comparative analysis of the N-glycome in CGL2-resistant C. *elegans* double mutants pmk-1; fut-8(op498) (upper trace) and pmk-1(km25); M03F8.4(op497) (middle trace) and the isogenic CGL2-hypersensitive single mutant strain pmk-1(km25) (lower trace).
**A. & B.** show the fluorescent curves of HPLC experiments of released and fluorescently labeled N-glycans. Upon enzymatic release (here the PNGase F resistant, but PNGase A sensitive glycome fraction is shown) and fluorescent labeling, N-glycans were separated by normal phase HPLC and analysed by mass spectrometry (**A**). Fractions at similar retention times (e.g. dashed rectangle) were further separated by reversed phase HPLC and the resulting pure glycans were analysed by mass spectrometry (MS) and for selected fractions by MS/MS (**B**).
**C**. shows the results o the structural characterization of a selected isolated peak. Peak A found in the *pmk-1* strain but not in the two double mutant strains was treated with β-1,4-galactosidase (β-1,4-Gal'ase) and α-fucosidase (α-Fuc'ase). The reaction products were analysed by reversed phase HPLC and MS and MS/MS. The lowest HPLC trace represents the glycan standard in glucose units (GU), Monosaccharides are represented as symbols: Man (dark gray circle), Gal (light grey circle), GlcNAc (square), Fuc (triangle), Hex (white circle).

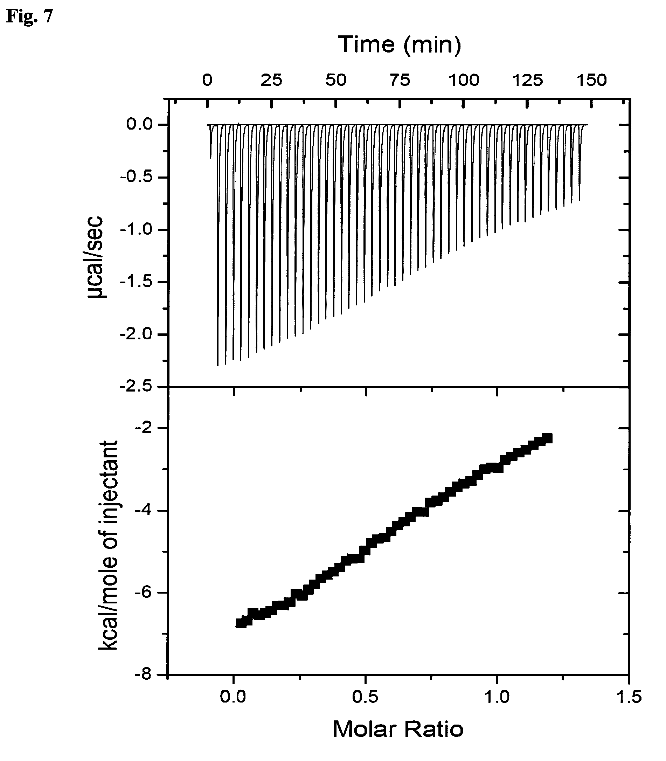

- **Fig. 7**: shows a graph of the *in vitro* binding of C. *cinerea* galectin CGL2 to chemically synthesized Gal-P-1,4-Fuc-α-1,6-GlcNAc-β-O-C₅H₁₀-NH₂ as determined by Isothermal Titration Calorimetry. The raw data is shown in the upper panel. Transformation of the data using the Microcal software yields the titration curve (lower panel), from which the dissociation constant (Kd = 86.9 ± 3.2 x 10-6 M) and the entropy of binding (TΔS = -3.31 kcal/mol) were calculated.
- **Fig. 8**: is a bar graph illustrating the RedA(CCL2)-sensitivity of various *C. elegans* glycosylation mutants. *C. elegans* mutants of the indicated genotypes were analysed for development from L1 to L4 on RedA-expressing *E. coli as* described in the below experimental procedures. Mutants not analyzed yet are indicated as n.d. See Table 1 below for glycosylation processes encoded by the various *C. elegans* genes.
- **Fig. 9**: is a bar graph illustrating the MOA-sensitivity test of various *C. elegans* glycosylation mutants. *C. elegans* mutants of the indicated genotypes were analysed for development from L1 to L4 on MOA-expressing *E. coli* as described in experimental procedures below. Mutants not analyzed yet are indicated as n.d. See Table 1 below for glycosylation processes encoded by the various *C. elegans* genes.
- **Fig. 10**: is a bar graph illustrating the AAL-sensitivity of various *C. elegans* glycosylation mutants. *C. elegans* mutants of the indicated genotypes were analysed for development from L1 to L4 on AAL-expressing *E. coli* as described in Experimental Procedures. See Table 1 below for glycosylation processes encoded by the various *C. elegans* genes.
- **Fig. 11**: relates to two graphs illustrating the carbohydrate-binding specificity of newly characterized fungal lectins *C. cinerea* RedA (CCL2) and S. *macrospora* TAP1 as well as the previously characterized *X*. *chrysenteron* lectin XCL. Recombinant proteins were fluorescently labeled and analyzed for binding to the glycan array by the Consortium of Functional Glycomics (CFG).
- **Fig. 12**: is a bar chart showing the toxicity of various fungal lectins towards *H. contortus.* The nematodes were analysed for development from L1 to L3 on lectin-expressing bacteria as described in the Experimental Procedures. The γ-coordinate is the fraction of worms reaching L3 in the presence of bacteria expressing the lectins on the X-coordinate. Fungal lectins analyzed included *C. cinerea* galectin CGL2 (Genbank AAF34732), galectin-related lectin CGL3 (Genbank ABD64675), lectin RedA (CCL2) (Genbank ACD88750), *Xerocomus chrysenteron* lectin XCL (Genbank AAL73235) and its ortholog TAP1 from *Sordaria macrospora* (Genbank CAH03681), *Aleuria aurantia* lectin AAL (Genbank BAA00451) and *Marasmius oreades* agglutinin MOA (Genbank AAL47680).

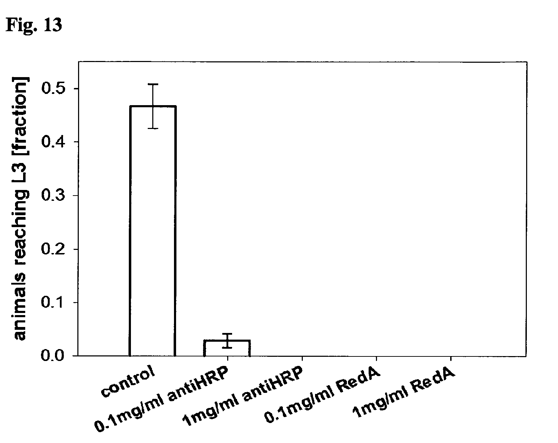

- **Fig. 13**: is a bar graph illustrating the effect of the commercially available IgG fraction of rabbit anti-peroxidase antiserum (Cat. No. 200-4138, Rockland Inc., USA; antiHRP) and purified RedA (CCL2) on the development of *H. contortus* L1 larvae to the L3 stage. The antibody and the lectin bind to the same epitope, Fuc-α-1,3-GlcNAc, present in the N-glycans of horseradish peroxidase (HRP) as well as of nematode glycoproteins, and were tested at two different concentrations. A non-glycan-binding rabbit IgG at a concentration of 1 mg/ml was used as control.

### Examples

The following examples describe (i) a method of the invention that allows for identifying helminths with at least one glycan mediating toxicity upon binding thereof to a glycan-binding polypeptide, (ii) a method for identifying helminthic gene targets involved in glycan-mediated toxicity using the previously identified helminth/glycan system that lead to the identification of the toxicity mediating glycans and (iii) a screening method for identifying anti-helminthic, preferably anti-nematode substances.

Furthermore, the examples describe the identification of fungal lectins that are toxic for model nematode *C. elegans* for which the toxicity-mediating glycoepitope/glycan/glycoconjugate has been identified. These medically useful lectins are
(i) CGL2 from *C. cinerea* which binds to Gal-beta-1,6-Fuc-alpha-1,6 on the Asn-linked GlcNAc of *C. elegans* N-glycan cores (see Figs. 1-7),
(ii) RedA (CCL2) from *C. cinerea* which binds to Fuc-alpha-1,3 on the Asn-linked GlcNAc of *C. elegans* N-glycan cores (Figs. 8 and 11),
(iii) MOA from *Marasmius oreades* which binds to Gal-alpha-1,3-GalNAc-beta-x,y on nematode glycosphingolipids (see Fig. 9),
(iv) XCL and TAP1 from *Xerocomus chrysenteron* and *Sordaria macrospora,* respectively, which bind to Gal-beta-1,3-GalNAc presumably on nematode O-glycans (see glycan array data [Fig. 11])
(v) AAL from *Aleuria aurantia* which binds terminal Fuc on a yet to be identified nematode glycoconjugate (see Fig. 10)

### General Experimental procedures

### Strains and cultivation conditions

*Escherichia coli* strains DH5a and BL21(DE3) were used for cloning and amplification of plasmids and bacterial expression of proteins, respectively. *E. coli* was cultivated on standard media as described in Sambrook, J., and Russell, D. W. (2001) Molecular Cloning: A Laboratory Manual, 3 ed., Cold Spring Harbor Laboratory. *Caenorhabditis elegans* strains were maintained on nematode growth media (NGM) and fed with *E. coli* strain OP50 as described in Stiernagle, T. (2006) WormBook, 1-11. The Bristol isolate N2 was used as the wild type strain. Strains *pmk-1(km25), bre-1(ye4), bre-2(ye31), bre-3(ye26), bre-4(ye27), bre-5(ye17), fut-1(ok892), fut-2(gk360), fut-2(ok509), fut-3(gk103), fut-4(gk111), fut-5(ok242), fut-6(ok475), fut-8(ok2558), gly-2(qa703), gly-12(is47), dpy-6(e14);gly-13(ok712), gly-14(id48), gly-20(ok826)* were obtained from the *Caenorhabditis* Genetics Center (CGC) at the University of Minnesota (USA). The strain carrying the two extrachromosomal constructs *frEx113*[*hsp::MosTransposase;P*_{*co*/}*₁₂::DsRed*] and *oxEx229*[*Mos1;P_{myo-2}::gfp*] was kindly provided by Jonathan Ewbank. For *Mos1-*mediated mutagenesis, we generated the strains *pmk-1(km25);frEx113* and *pmk-1(km25);oxEx229.* Strains resulting from *Mos1*-mediated mutagenesis and subsequent outcrossing were *pmk-1(km25); M03F8.4(op497), pmk-1(km25); fut-8(op498), pmk-1 (km25); ger-1(op499), pmk-1 (km25);gly-13(op507)* and *pmk-1 (km25);bre-1 (op509).*

### Cloning and expression

Plasmids for bacterial expression of fungal lectins were constructed by amplifying the respective open reading frame from cDNA or available plasmids and ligating the resulting fragment into pET24a (Invitrogen) using introduced restriction sites. Expression of CGL2 and CGL2(W72G) in liquid culture was performed as described for CGL3 in Walti, M. A. et al. (2008) J. Mol. Biol. 379, 146-159. For the *C. elegans* bioassays, 300 µl of an overnight culture of the respective BL21(DE3) transformants were spread on NGM-plates containing 1 mM isopropyl-β-D-thiogalactoside (IPTG) and 50 µg/m) Kanamycin and incubated overnight at 23°C before addition of the nematodes. Lectin expression was verified by separating whole cell extracts of induced BL21(DE3)-transformants on Coomassie blue-stained SDS-polyacrylamide gels and immunoblotting using specific antisera if available (data not shown).

### CGL2 purification and labelling

Bacterial cell pellets were resuspended in ice-cold phosphate-buffered saline (PBS) [30 mM Na-phosphate pH 7.3, 150 mM NaCl] containing 1 mM phenylmethylsulfonyl fluoride and ruptured using a French press. Cell debris was removed in two consecutive steps of centrifugation at 12000 g for 15 min and 27000 g for 30 min. The supernatant was incubated with lactosyl-sepharose at 4 °C for 1 h and CGL2 was finally eluted at room temperature in PBS containing 200 mM lactose. After size exclusion chromatography on Superose 6 10/300 GL (GE Healthcare) equilibrated in PBS, fractions containing the protein were pooled and concentrated using an Amicon Ultra-4 centrifugal filter device (Millipore) with a molecular weight cutoff of 10 kDa. Protein concentration was calculated by measuring the absorbance at 280 nm, assuming a relation of 1.25 mg ml⁻¹ to 1 unit absorbance at 280 nm for a path length of 1 cm. Conjugation of purified CGL2 to tetramethylrhodamin (TAMRA) (Molecular Probes) was performed as described in Walser, P. J. et al. (2005) Fungal Genet Biol 42, 293-305.

### Light microscopy of C. elegans

For general worm handling, a Leica MZ 12.5 stereomicroscope was used. To select double-array carrying worms *(pmk-1(km25);oxEx229;frEx113),* a Leica MZ 16 FA stereomicroscope equipped with appropriate filtersets (DsRed and GFP filter) was used. Pictures were taken with a Nikon Coolpix 990 digital camera.

For DIC and fluorescence microscopy, worms were placed on 2% agarose pads in M9 (Sambrook & Russell, 2001), anaesthesized with levamisole (3-5 mM) (Sigma) and mounted under a coverslip for observation using a Leica DM-RA or Zeiss Axiovert 200 microscope equipped with DIC (Nomarski) optics and standard epifluorescence with a DsRed filterset for detection of TAMRA. Pictures were taken with a Hamamatsu ORCA-ER camera. Images were false-coloured using OpenLab software.

For the *in situ* localization of the CGL2-ligand, L4 staged *C. elegans* were placed in wells containing S-medium (Sulston & Hodgkin, 1988), BL21(DE3) E. *coli* harbouring empty Kan^{R}-vector, kanamycin and chloramphenicol at 30 µg/ml each and TAMRA-labeled CGL2 at 100 µg/ml. After 24 hrs, worms were transferred to standard OP50 plates and screened for TAMRA fluorescence 2 h thereafter.

### Electron microscopy of C. elegans

For electron microscopic examination, worms were prepared by a described two-step chemical fixation (Hall, 1995). Fixation and slicing of the samples was kindly carried out by Garry Barmettler at the Center for Microscopy and Image Analysis (University of Zurich, Switzerland). The samples were examined using a Philips CM100 transmission electron microscope equipped with a side mounted digital camera (Gatan).

### Example 1 - C. elegans toxicity assays

### Plate assay

A plate assay was devised to examine the toxicity of wild type and mutant CGL2 towards *C. elegans.* NGM plates were seeded with *E. coli* BL21 (DE3) expressing either wild type CGL2 or mutant CGL2(W72G) as described above. As a control, plates were seeded with *E. coli* BL21(DE3) containing the vector pET24a. The plates were incubated overnight at 23°C and seeded with synchronized populations of *C. elegans* (see Barrows, B. D.et al. (2006) Methods Enzymol 417, 340-358) for the different toxicity assays:

### Phenotypic analysis

For phenotypic analysis of the toxic effect of CGL2 towards *C. elegans,* L4 animals were seeded onto the plates and examined after 24 h at 23°C.

### Developmental assay

Quantitative data on the effect of CGL2 on *C. elegans* development was acquired by placing 50 to 100 newly hatched L1 larvae of the indicated genotypes on the plates. After 72 h, the fraction of animals that reached L4 stage was determined.

### Brood size assay

The effect of CGL2 on *C. elegans* reproduction was assayed by picking individual L4 wild type hermaphrodites onto plates. Thereafter, the mothers were transferred to new plates daily until the mother either stopped producing offspring or died. The progeny of the previous plate were counted the next day. The number of progeny from the various plates were added up to give the final brood size.

### Example 2 - Determination of the CGL2 Minimal Inhibitory Concentration (MIC)

The MIC was defined as the concentration of toxin at which >50% of the animals fail to reach larval stage 4 (L4) within 96 h in liquid culture. 20 L1 staged *C. elegans* wild type worms were placed in wells containing S-medium (see Sulston & Hodgkin (1988) Methods. in The nematode Caenorhabditis elegans (Wood, W. B. ed.), Cold Spring Harbor Laboratory Press, New York. pp 587-606), *E. coli* BL21 containing empty vector pET24a with a Kan^{R} gene as a food source, kanamycin and chloramphenicol (30 µg/ml each) and purified CGL2 protein in the concentrations indicated. After 96 h, the worms were transferred to NGM plates and the number of worms that reached L4 stage was determined.

### Example 3 - Hypersensitivity test

10 L4 stage *C. elegans* of the indicated genotypes were transferred to CGL2 plates. The plates were checked for surviving animals every 24 h. A worm was considered dead when it did not react to touching with a worm pick.

### Example 4 - Screen for CGL2-resistant C. elegans mutants using Mos1 insertional mutagenesis

*Mos1* insertional mutagenesis was in principle performed as published in Boulin and Bessereau(2007) Nat. Protoc. 2, 1276-1287. The extrachromosomal arrays frEx113, which carries the *Mos1* transposase under the control of a heat-shock promoter, and *oxEx229,* which carries multiple copies of the substrate *Mos1* transposon were used. The two extrachromosomal constructs were crossed into CGL2-hypersensitive *pmk-1(km25)* worms to generate the two starting strains *pmk-1(km25); frEx113* and *pmk-1(km25);oEx229* for the screen. To generate double-array carrying animals, *pmk-1(km25); oxEx229* males were crossed to L4 *pmk-1 (km25); frEx113* hermaphrodites. Progeny containing both arrays, recognized by the concurrent expression of GFP in the pharynx (*P_{myo-2}::gfp,* contained in *oxEx229*) and DsRed in the coelomocytes (*P*_{*co*/}*₁₂::DsRed,* contained in *frEx113*), were propagated for approximately six generations before subjecting them to heat-shock. Several hundred double-transgenic animals per mutagenesis round were subjected to heat-shock for 1 h at 33°C, 1 h at 20°C, and 1 h at 33°C and then allowed to recover overnight at 20°C. P₀ were distributed to 90 mm NGM plates and eggs were collected 12-40 h after heat shock. P₀ were then removed. After 3 days, gravid F₁ were washed off the plates in M9 buffer and F₂ eggs were isolated as described in Stiernagle, T. (2006) WormBook, 1-11. The synchronized population of F₂ L1 animals was distributed on 30 plates containing CGL2-expressing *E. coli* BL21 (DE3). After 3 to 7 days, these plates were screened for CGL2-resistant animals that had reached adulthood. Each plate with resistant animals was treated as an individual hit to avoid redundant *Mos1* insertions. Candidate worms were propagated on CGL2-expressing *E. coli* to confirm the resistance phenotype. Once resistance was confirmed, mutants that had lost the extrachromosomal *Mos1*-bearing array were outcrossed 2 to 6 times before assaying for the presence of *Mos1* elements and trying to locate the site of insertion. For those mutants that still contained a *Mos1* element, we determined the insertion site through inverse PCR on worm lysates as published in Boulin and Bessereau (2007) Nat. Protoc. 2, 1276-1287.

In total, approximately 500'000 haploid genomes were screened. The transposition efficiency was measured as 50%. In total, 14 CGL2-resistant worms were isolated. Only 5 of them, *pmk-1(km25); M03F8.4(op497), pmk-1(km25); fut-8(op498), ger-1(op499); pmk-1(km25), pmk-1(km25); gly-13(op507)* and *pmk-1 (km25); bre-1 (op509),* contained the *Mos1* transposon insertion.

### Example 5 - Isolation of C. elegans N-glycans

*C. elegans* strains *pmk-1(km25), pmk-1(km25); M03F8.4(op497)* and *pmk-1(km25); fut-8(op498)* were grown for 5 days at room temperature in liquid culture with *E. coli* OP50 and afterwards separated from bacteria and debris by 30 % (w/v) sucrose gradient centrifugation (see Stiernagle, T. (2006) WormBook, 1-11). *N*-glycan preparation was performed as previously published in Poltl et al. (2007) Febs J. 274, 714-726, by enzymatic release of glycans from partially purified glycopeptides using peptide-N-glycanase (PNGase) F and subsequently PNGase A in order to separate core α1,3-fucosylated glycans from other core fucosylated glycans. Briefly, approximately 3 g of worms (wet weight) were boiled prior to grinding. The extract was adjusted to contain 5 % (v/v) formic acid and incubated with 3 mg pepsin (Sigma) at 37°C overnight. After centrifugation at 15'000 g for 15 min, the supernatant was applied to 15 ml Dowex AG WX2 equilibrated with 2 % acetic acid. The glycopeptides were eluted with ammonium acetate (0.6 M, pH 6). Orcinol-positive fractions were pooled and lyophilized overnight. The samples were then desalted by application to a Sephadex G25 column and eluted with 1 % acetic acid. The orcinol-positive fractions were again pooled and lyophilized. The samples were dissolved in 250 µl water. After heat treatment at 95°C for 5 min to inactivate residual pepsin, the samples were cooled prior to addition of 250 µl ammonium carbonate buffer pH 8 and 3 U PNGase F (Roche) and incubated overnight at 37 °C. The samples were then acidified with 400 µl 10% acetic acid and applied to 5 ml Dowex AG WX2. The unretained free glycans were lyophilized and dried for subsequent fluorescent labeling whereas the retained Orcinol positive fractions eluting with ammonium acetate (0.6 M, pH 6) were desalted as above, dissolved in ammonium acetate buffer (50 mM, pH 5) and treated with PNGase A (0.6 mU) overnight at 37°C. Again the samples were acidified with 400 µl 10 % acetic acid and applied to 5 ml Dowex AG WX2. The unretained free glycans were lyophilized and also dried for subsequent fluorescent labeling.

### Example 6 - Labelling and structural analysis of C. elegans N-glycans

Fluorescent labelling of the N-glycans was performed as previously described **(where ?)** using 2-amino-pyridine (PA). Complete N-glycomes of either PNGase A or F released and pyridylaminated glycans were fractionated by 2D-HPLC using a Shimadzu HPLC system (consisting of a SCL-10A controller, two LC10AP pumps and a RF-10AXL fluorescence detector controlled by a personal computer using Class-VP software (V6.13SP2)) at room temperature and fluorescence detection (excitation at 310 or 320 nm, emission detected at 380 or 400 nm). The N-glycans were first fractionated on a normal phase HPLC (Tosoh TSK gel Amide-80, 4.6 x 250 mm, 5 µm; flow 1 ml/min, elution: 5 min isocratic 71.3 % MeCN, 10 min gradient from 71.3 % to 61.8 % MeCN, 25 min isocratic 61.8 % MeCN, 15 min 61.8 % to 54.2 % MeCN using ammonium formate (10 mM, pH 7) as buffer). The fractions were lyophilized and further fractionated on reversed phase HPLC (Hypersil ODS C-18; 4 x 250 mm, 5 µm; flow 1.5 ml/min, gradient of 0 - 30 % MeOH over 30 min using ammonium formate (0.1 M, pH 4) as buffer). HPLC chromatograms were visualized using the opensource program PLOT (Version 0.997 by Wesemann and Thijsse). Each fraction was subjected to monoisotopic MALDI-TOF MS using a Bruker Ultraflex TOF/TOF with 2,5-dihydroxybenzoic acid as matrix. In general, all fractions with fucose containing N-glycans were subjected to MS/MS to elucidate their composition. A peptide standard mixture (Bruker) was used for external calibration. MS data were analysed using Bruker software and the mMass V2.4 software package (see Strohalm, M. et al., M. (2008) Rapid Commun Mass Spectrom 22, 905-908).
Selected isolated N-glycans were examined for the presence of either terminal β-gala - ctosides by treatment with *Aspergillus oryzae* β1,4-galactosidase (27 mU, 50 mM sodium citrate, pH 4.5) (see Gutternigg, M. et al. (2007) J. Biol. Chem. 282, 27825-27840) for 2 days at 37°C or terminal α-fucosides by use of bovine kidney α-fucosidase (Sigma, 15 mU, 50 mM ammonium acetate, pH 5). Digestion products were subsequently analysed for altered structural characteristics by RP-HPLC (see above) and MALDI-TOF MS.

### Example 7 - Isothermal titration microcalorimetry (ITC) measurements

Experiments were performed with a VP-ITC isothermal titration calorimeter (Microcal, Massachusetts, USA) at 25 °C using purified CGL2 (300 µM in TBS). Gal-β-1,4-Fuc-α-1,6-GlcNAc-β-O-C5H10-NH2 was dissolved in TBS at a concentration of 1.6 mM corrected based on purity estimate by ¹H-NMR (ca. 50-60%). The ligand was titrated into a cell containing the protein solution in 48 injections of 6 µl preceded by a single injection of 2 µl. All injections were performed at intervals of 3 min while stirring at 270 rpm. Experimental data were fitted to a theoretical titration curve using the MCS-ORIGIN software supplied by Microcal. The number of binding sites (N = 0.977 ± 0.004), the association constant (Ka = 1.15 ± 0.044 x 104 M-1) and enthalpy change (ΔH = -8.85 ± 0.1 kcal/mol) were obtained using a model of one ligand-binding site per protein monomer.

### Example 8 - Haemonchus contortus toxicity assays

The toxicity towards the above-mentioned parasitic nematode was evaluated in a liquid assay using 96-well cell culture plates. *H*. *contortus* eggs were isolated from feces of an infected sheep and hatched on water-agar plates containing Kanamycin (50 µg/ml) and Amphotericin B (2.5µg/ml). Approximately 30 L1 larvae were collected and transferred to each well, in a total volume of 150 µl 0.1x Earls solution. As food source, either lectin expressing *E*. *coli* BL21(DE3) cells or *E*. *coli* OP50 (OD₆₀₀ = 1) in combination with purified antibody or lectin at different concentrations were added. Plates were incubated at 28°C in the dark, and percentage of larvae that developed to L3 were determined after 7 days.

**Table 1: Compilation of C. elegans glycosylation mutants. Functions of encoded enzymes are taken from www.wormbase.org.**

| **Gene(s)** | **Glycosylation** |
|---|---|
| *bre-1, ger-1* | Biosynthesis of GDP-fucose, the donor substrate for fucosyltransferases |
| *fut-1* | Fucosyltransferase responsible for biosynthesis of core α-1,3-fucose on Asn-linked GlcNAc of *C*. *elegans* N-glycans |
| *fut-8* | Fucosyltransferase responsible for biosynthesis of core α-1,3-fucose on Asn-linked GIcNAc of *C*. *elegans* N-glycans |
| *M03F8.4 (galt-1)* | Galactosyltransferase responsible for biosynthesis of core Gal-β-1,4-Fuc-α-1,6 on Asn-linked GlcNAc of *C*. *elegans* N-glycans, identified in forward |
| | genetic screen for CGL2-resistant *C*. *elegans* mutants (Fig. 3) |
| *bre-2, bre-3, bre-4, bre-5* | Glycosyltransferases responsible for biosynthesis of diverse C. *elegans* glycosphingolipids |
| *fut-2* | α-1,2-fucosyltransferase of unknown acceptor specificity |
| *fut-3* to *fut-6* | Fucosyltransferases of unknown acceptor specificity |
| *gly-12, gly-13, gly-14* | GlcNAc-transferaseses responsible for biosynthesis of GlcNAc-β-1,2 on Man-α-1,3 of paucimannose-type C. elegans N-glycans (GnTI), a modification required for biosynthesis of complex *C*. *elegans* N-glycans including core modifications |
| *gly-2* | GlcNAc-transferasese responsible for biosynthesis of GlcNAc-β-1,6 on Man-α-1,6 of paucimannose-type *C*. *elegans* N-glycans (GnTV) |
| *gly-20* | GlcNAc-transferasese responsible for biosynthesis of GlcNAc-β-1,2 on Man-α-1,6 of paucimannose-type *C*. *elegans* N-glycans (GnTII) |

The invention has been described with the emphasis upon preferred embodiments and illustrative examples. However, it will be obvious to those of ordinary skill in the art that variations of the preferred embodiments may be used and that it is intended that the invention may be practiced otherwise than as specifically described herein. Moreover, as the foregoing examples are included for purely illustrative purposes, they should not be construed to limit the scope of the invention in any respect. Accordingly, this invention includes all modifications encompassed within the spirit and scope of the invention as defined by the claims appended hereto.

## Claims

1. Glycan-binding polypeptides for use in treating and/or preventing helminthic infections, wherein the glycan-binding polypeptides bind N-glycans, O-glycans or lipoglycans, wherein the
I. N-glycans are fucoside-containing oligo/polysaccharides and/or glycoconjugates, preferably
(i) D-galacto-pyranosyl-β-1,4-L-fucopyranosyl-containing oligo/polysaccharides and/or glycoconjugates, more preferably D-galacto-pyranosyl-β-1,4-L-fucopyranosyl-α-1,6-GlcNAc-containing oligo/polysaccharides and/or glycoconjugates, more preferably GnGnF⁶Gal- and/or MMF⁶Gal-containing oligosaccharides and glycoconjugates, or
(ii) L-fucopyranosyl-α-1,3- containing oligo/polysaccharides and/or glycoconjugates, more preferably L-fucopyranosyl-α-1,3-GlcNAc-containing oligo/polysaccharides and/or glycoconjugates, more preferably GnGnF³- and/or MMF³-containing oligosaccharides and glycoconjugates;
II. O-glycans are galactoside-containing oligo/polysaccharides and/or glycoconjugates, preferably D-galacto-pyraraosyl-β-1,3-containing oligo/polysaccharides and/or glycoconjugates, more preferably D-galactopyranosyl-β-1,3-N-acetyl-D-gaiactasamino-pyranosyl-containing oligo/polysaccharides and/or glycoconjugates, most preferably D-galactopyranosyl-β-1,3-N-acetyl-D-galactosamino-pyranosyl-O-Ser/Thr containing oligo/polysaccharides and/or glycoconjugates;
III. lipoglycans are galactoside-containing oligo/polysaccharides and/or glycoconjugates, preferably D-galacto-pyranosyl-α-1,3-containing oligo/polysaccharides and/or glycoconjugates, more preferably D-galactopyranosyl-α-1,3-N-acetyl-D-galactosamino-pyranosyl-containing oligo/polysaccharides and/or glycoconjugates, most preferably D-galactopyranosyl-α-1,3-N-acetyl-D-galactosamino-pyranosyl-β-1,4-N-acetyl-D-glucosamino-pyranosyl-containing oligo/polysaccharides and/or glycoconjugates.

2. Glycan-binding polypeptides for use according to claim 1 , wherein the polypeptides bind to at least one nematode glycan, preferably produced by nematodes selected from the family Trichostrongylidae, preferably *Haemonchus contortus, Trichostrongylus colubriformis, Teladorsagia circumcincta, Cooperia oncophora, Nematodirus battus*, *Ostertagia* leptospicularis, *Chabertia ovina, Oesophagostomum dentatum,* and nematode species *Trichinella spiralis, Trichuris trichuria, Angiostrongylus vasorum, Ancylostoma caninum, Ancylostoma duodenale*, *Ancylostoma ceylanicum*, *Necator americanus*, *Dictyocaulus* spp., *Ascaris lumbricoldes*, *Ascaris suum, Wuchereria bancrofti*, *Brugia malayi, Loa loa, Enterobius vermicularis, Dirofilaria immitis, Onchocerca volvulus and Strongyloides stercoralis.*

3. Glycan-binding polypeptides for use according to any one of claims 1 or 2, wherein the polypeptides are selected from the group consisting of antibodies, fragments or functional derivatives of antibodies and antibody-like binding proteins.

4. Glycan-binding polypeptides for use according to any one of claims 1 to 3, wherein the polypeptides are polyvalent and thus bind to at least two or more glycans.

5. Glycan-binding polypeptides for use according to claim 1 to 4, wherein the glycan-binding polypeptides are selected from the group of fungal lectins, preferably lectins from *Coprinopsis cinerea, Xerocomus chrysenteron, Marasmius oreades, Aleuria aurantia* or *Sordaria macrospora,* more preferably lectins selected from the group consisting of CGL1, CGL2, RedA (CCL2), CCL1, XCL, MOA, AAL and TAP1.

6. Glycan-binding polypeptides for use according to any one of claims 1 to 5 , wherein the glycan-binding polypeptides bind N-glycans and the heliminthic infection is a nematode infection.

7. Glycan-binding polypeptides for use according to any of claims 1 to 6, wherein the helminthic, preferably nematode infection is an infection resulting from a helminth selected from the family Trichostrongylidae, preferably *Haemonchus contortus, Trichostrongylus colubriformis, Teladorsagia circumcincta, Cooperia oncophora, Nematodirus battus,* Ostertagia leptospicularis, *Chabertia ovina, Oesophagostomum dentatum*, and nematode species *Trichinella spiralis, Trichuris trichuria, Angiostrongylus vasorum, Ancylostoma caninum, Ancylostoma duodenale*, *Ancylostoma ceylanicum, Necator americanus, Dictyocaulus* spp., *Ascaris lumbricoides, Ascaris suum*, *Wuchereria bancrofti, Brugia malayi, Loa loa, Enterobius vermicularis, Dirofilaria immitis, Onchocerca volvulus and Strongyloides sfercoralis.*

8. Pharmaceutical composition comprising at least one glycan-binding polypeptide for use in treating and/or preventing a helminthic, preferably nematode infection wherein the glycan-binding polypeptide binds N-glycans, O-glycans or lipoglycans, wherein the
I. N-glycans are fucoside-containing oligo/polysaccharides and/or glycoconjugates, preferably
(i) D-galacto-pyranosyl-β-1,4-L-fucopyranosyl-containing oligo/polysaccharides and/or glycoconjugates, more preferably D-galacto-pyranosyl-β-1,4-L-fucopyranosyl-α-1,6-GlcNAc-containing oligo/polysaccharides and/or glycoconjugates, more preferably GnGnF⁶Gal- and/or MMF⁶Gal-containing oligosaccharides and glycoconjugates, or
(ii) L-fucopyranosyl-α-1,3- containing oligo/polysaccharides and/or glycoconjugates, more preferably L-fucopyranosyl-α-1,3-GlcNAc-containing oligo/polysaccharides and/or glycoconjugates, more preferably GnGnF³- and/or MMF³-containing oligosaccharides and glycoconjugates;
II. O-glycans are galactoside-containing oligo/polysaccharides and/or glycoconjugates, preferably D-galacto-pyranosyl-β-1,3-containing oligo/polysaccharides and/or glycoconjugates, more preferably D-galactopyranosyl-β-1,3-N-acetyl-D-galactosamino-pyranosyl-containing oligo/polysaccharides and/or glycoconjugates, most preferably D-galactopyranosyl-β-1,3-N-acetyl-D-galactosamino-pyranosyl-O-Ser/Thr containing oligo/polysaccharides and/or glycoconjugates;
III. lipoglycans are galactoside-containing oligo/polysaccharides and/or glycoconjugates, preferably D-galacto-pyranosyl-α-1,3-containing oligo/polysaccharides and/or glycoconjugates, more preferably D-galactopyranosyl-α-1,3-N-acetyl-D-galactosamino-pyranosyl-containing oligo/polysaccharides and/or glycoconjugates, most preferably D-galactopyranosyl-α-1,3-N-acetyl-D-galactosamino-pyranosyl-β-1,4-N-acetyl-D-glucosamino-pyranosyl-containing oligo/polysaccharides and/or glycoconjugates.

9. Composition for use according to claim 8, wherein the glycan-binding polypeptide binds to at least one nematode glycan produced by nematodes selected from the family Trichostrongylidae, preferably *Haemonchus contortus, Trichostrongylus colubriformis, Teladorsagia circumcincta, Cooperia oncophora, Nematodirus battus*, Ostertagia leptospicularis, *Chabertia ovina, Oesophagostomum dentatum*, and nematode species *Trichinella spiralis, Trichuris trichuria, Angiostrongylus vasorum, Ancylostoma caninum*, *Ancylostoma duodenale*, *Ancylostoma ceylanicum, Necator americanus*, *Dictyocaulus* spp., *Ascaris lumbricoides, Ascaris suum, Wuchereria bancrofti*, *Brugia malayi, Loa loa, Enterobius vermicularis*, *Dirofilaria immitis, Onchocerca volvulus and Strongyloides stercoralis.*

10. Composition for use according to any of claims 8 or 9, wherein the polypeptides are selected from the group consisting of antibodies, fragments or functional derivatives of antibodies and antibody-like binding proteins.

11. Composition for use according to any of claims 8 to 10, wherein the polypeptides are polyvalent and thus bind to at least two or more glycans.

12. Composition for use according to any of claims 8 to 11, wherein the polypeptides are selected from the group of fungal lectins, preferably lectins from *Coprinopsis cinerea, Xerocomus chrysenteron, Marasmius oreades, Aleuria aurantia* or *Sordaria macrospora*, more preferably lectin CGL1, CGL2, RedA (CCL2), CCL1 XCL, MOA, AAL or TAP1.

13. Composition for use according to any one of claims 8 to 12, wherein the glycan-binding polypepides are displayed on the surface of bacterial cells, preferably enterobacteria, more preferably human enterobacterial cells, most preferably *Escherichia coli* or *Salmonella typhimurium*.

14. Food product comprising isolated glycan-binding polypeptides according to any one of claims 1 to 7 for use in treating and/or preventing a helminthic, preferably nematode infection.

15. Animal feed comprising isolated glycan-binding polypeptides according to any one of claims 1 to 7 for use in treating and/or preventing a helminthic, preferably nematode infection.

## Patentansprüche

1. Glykan-bindende Polypeptide zur Verwendung in der Behandlung und/oder Vorbeugung von helminthischen Infektionen, wobei die Glykan-bindenden Polypeptide N-Glykane, O-Glykane oder Lipoglykane binden, wobei die
I. N-Glykane Fucosid-enthaltende Oligo-/Polysaccharide und/oder Glycokonjugate sind, vorzugsweise
(i) D-galacto-pyranosyl-β-1,4-L-fucopyranosyl-enthaltende Oligo-/Polysaccharide und/oder Glycokonjugate, mehr bevorzugt D-Galacto-pyranosyl-β-1,4-L-fucopyranosyl-α-1,6-GlcNAc-enthaltende Oligo-/Polysaccharide und/oder Glycokonjugate, mehr bevorzugt GnGnF⁶Gal- und/oder MMF⁶Gal-enthaltende Oligosaccharide und Glycokonjugate, oder
(ii) L-Fucopyranosyl-α-1,3-enthaltende Oligo-/Polysaccharide und/oder Glycokonjugate, mehr bevorzugt L-Fucopyranosyl-α-1,3-GlcNAc-enthaltende Oligo-/Polysaccharide und/oder Glycokonjugate, mehr bevorzugt GnGnF³- und/oder MMF³-enthaltende Oligosaccharide und Glycokonjugate;
II. O-Glycane Galactosid-enthaltende Oligo-/Polysaccharide und/oder Glycokonjugate sind, vorzugsweise D-Galacto-pyranosyl-β-1,3-enthaltende Oligo-/Polysaccharide und/oder Glycokonjugate, mehr bevorzugt D-Galacto-pyranosyl-β-1,3-N-acetyl-D-galactosamino-pyranosyl-enthaltende Oligo-/Polysaccharide und/oder Glycokonjugate, am meisten bevorzugt D-Galacto-pyrarosyl-β-1,3-N-acetyl-D-galactosamino-pyranasyl-O-Ser/Thr-enthaltende Oligo-/Polysaccharide und/oder Glycokonjugate;
III. Lipoglykane Galactosid-enthaltende Oligo-/Polysaccharide und/oder Glycokonjugate sind, vorzugsweise D-Galacto-pyranosyl-α-1,3-enthaltende Oliga-/Polysaccharide und/oder Glycokonjugate, mehr bevorzugt D-Galacto-pyranosyl-α-1,3-N-acetyl-D-galactosamino-pyranosyl-enthaltende oligo-/Polysaccharide und/oder Glycokonjugate, am meisten bevorzugt D-Galacto-pyranosyl-α-1,3-N-acetyl-D-galactosamino-pyranosyl-β-1,4-N-acetyl-D-glucosamino-pyranosyl-enthaltende Oligo-/Polysaccharide und/oder Glycokonjugate.

2. Glykan-bindende Polypeptide zur Verwendung gemäss Anspruch 1, wobei die Polypeptide an mindestens ein Nematodenglykan binden, vorzugsweise hergestellt von Nematoden ausgewählt aus der Familie Trichostrongylidae, vorzugsweise *Haemonchus contortus, Trichostrongylus colubriformis, Teladorsagia circumcincta, Cooperia oncophora, Nematodirus battus*, *Ostertagia leptospicularis, Chabertia ovina, Oesophagostomum dentatum,* und von den Nematodenspezies *Trichinella spiralis, Trichuris trichuria, Angiostrongylus vasorum, Ancylostoma caninum, Ancylostoma duodenale, Ancylostoma ceylanicum, Necator americanus, Dictyocaulus* spp., *Ascaris lumbricoides, Ascaris suum, Wuchereria bancrofti, Brugia malayi, Loa loa, Enterobius vermicularis, Dirofilaria immitis, Onchocerca volvulus und Strongyloides stercoralis.*

3. Glykan-bindende Polypeptide zur Verwendung gemäss irgendeinem der Ansprüche 1 oder 2, wobei die Polypeptide ausgewählt sind aus der Gruppe bestehend aus Antikörpern, Fragmenten oder funktionellen Derivaten von Antikörpern und antikörperartigen Bindungsproteinen.

4. Glykan-bindende Polypeptide zur Verwendung gemäss irgendeinem der Ansprüche 1 bis 3, wobei die Polypeptide polyvalent sind und daher an mindestens zwei oder mehr Glykane binden.

5. Glykan-bindende Polypeptide zur Verwendung gemäss irgendeinem der Ansprüche 1 bis 4, wobei die Glykan-bindenden Polypeptide ausgewählt sind aus der Gruppe der Pilzlektine, vorzugsweise Lektine aus *Coprinopsis cinerea, Xerocomus chrysenteron, Marasmius oreades, Aleuria aurantia* oder *Sordaria macrospora,* mehr bevorzugt Lektine ausgewählt aus der Gruppe bestehend aus CGL1, CGL2, RedA (CCL2), CCL1, XCL, MOA, AAL und TAP1.

6. Glykan-bindende Polypeptide zur Verwendung gemäss irgendeinem der Ansprüche 1 bis 5, wobei die Glykan-bindenden Polypeptide N-Glykane binden und die helminthische Infektion eine Nematodeninfektion ist.

7. Glykan-bindende Polypeptide zur Verwendung gemäss irgendeinem der Ansprüche 1 bis 6, wobei die helminthische Infektion, vorzugsweise Nematodeninfektion, eine Infektion ist, die durch einen Helminthen verursacht wird, ausgewählt aus der Familie Trichostrongylidae, vorzugsweise *Haemonchus contortus, Trichostrongylus colubriformis, Teladorsagia circumcucta, Cooperio ancophora, Nematodirus battus, Ostertagia leptospicularis, Chabertia ovina, Oesaphagostomum dentatum,* und den Nematodenspezies *Trichinella spiralis, Trichuris trichuria, Angiostrongylus vasorum, Ancylostoma caninum, Ancylostoma duodenale, Ancylostoma ceylanicum, Necator americanus, Dictyocaulus* spp., *Ascaris lumbricoides, Ascaris suum, Wuchereria bancrofti*, *Brugia malayi, Loa loa, Enterobius vermicularis, Dirofilaria immitis, Onchocerca volvulus und Strongyloides stercoralis*.

8. Pharmazeutische Zusammensetzung umfassend mindestens ein Glykan-bindendes Polypeptid zur Verwendung in der Behandlung und/oder Vorbeugung einer heiminthischen Infektion, vorzugsweise einer Nematodeninfektion, wobei das Glykan-bindende Polypeptid N-Glykane, O-Glykane oder Lipoglykane bindet, wobei die
I. N-Glykane Fucosid-enthaltende Oligo-/Polysaccharide und/oder Glycokonjugate sind, vorzugsweise
(i) D-galacto-pyranosyl-β-1,4-L-fucopyranosyl-enthaltende Oligo-/Polysaccharide und/oder Glycokonjugate, mehr bevorzugt D-Galacto-pyranosyl-β-1,4-1-fucopyranosyl-α-1,6-GlcNAc-enthaltende Oligo-/Polysaccharide und/oder Glycokonjugate, mehr bevorzugt GnGnF⁶Gal- und/oder MMF⁶Gal-enthalfende Oligosaccharide und Glycokonjugate, oder
(ii) L-Fucopyranosyl-α-1,3-enthaltende Oligo-/Polysaccharide und/oder Glycokonjugate, mehr bevorzugt L-Fucopyranosyl-α-1,3-GlcNAc-enthaltende Oligo-/Polysaccharide und/oder Glycokonjugate, mehr bevorzugt GnGnF³- und/oder MMF³-enthaltende Oligosaccharide und Glycokonjugate;
II. O-Glycane Galactosid-enthaltende Oligo-/Polysaccharide und/oder Glycokonjugate sind, vorzugsweise D-Galacto-pyranosyl-β-1,3-enthaltende Oligo-/Polysaccharide und/oder Glycokonjugate, mehr bevorzugt D-Galacto-pyranosyl-β-1,3-N-acetyl-D-gslactosamino-pyranosyl-enthaltende Oligo-/Polysaccharide und/oder Glycokonjugate, am meisten bevorzugt D-Galacto-pyranosyl-β-1,3-N-acetyl-D-galactosamino-pyranosyl-O-Ser/Thr-enthaltende Oligo-/Polysaccharide und/oder Glycokonjugate;
III. Lipoglykane Galactosid-enthaltende Oligo-/Polysaccharide und/oder Glycokonjugate sind, vorzugsweise D-Galacto-pyranosyl-α-1,3-enthaltende Oligo-/Polysaccharide und/oder Glycokonjugate, mehr bevorzugt D-Galacto-pyranosyl-α-1,3-N-acetyl-D-galactosamino-pyranosyl-enthaltende Oliga-/Palysaccharide und/ader Glycokanjugate, am meisten bevorzugt D-Galacto-pyranosyl-α-1,3-N-acetyl-D-galactosamino-pyranosyl-β-1,4-N-acetyl-D-glucosamino-pyranosyl-enthaltende Oligo-/Polysaccharide und/oder Glycokonjugate.

9. Zusammensetzung zur Verwendung gemäss Anspruch 8, wobei das Glykan-bindende Polypeptid an mindestens ein Nematodenglykan bindet, hergestellt von Nematoden ausgewählt aus der Familie Trichostrongylidae, vorzugsweise *Haemonchus contortus, Trichostrongylus colubriformis, Teladorsagia circumcincta, Cooperia oncophora, Nematodirus battus, Ostertagia leptospicularis, Chabertia ovina, Oesaphagostomum dentatum,* und aus den Nematodenspezies *Trichinella spiralis, Trichuris trichuria, Angiostrongylus vasorum, Ancylostoma caninum, Ancylostoma duodenale, Ancylostoma ceylanicum, Necator americanus, Dictyocaulus* spp., *Ascaris lumbricoides, Ascaris suum, Wuchereria bancrofti*, *Brugia malayi, Loa loa, Enterobius vermicularis, Dirofilaria immitis, Onchocerco valvulus und Strongyloides stercoralis.*

10. Zusammensetzung zur Verwendung gemäss irgendeinem der Ansprüche 8 oder 9, wobei die Polypeptide ausgewählt sind aus der Gruppe bestehend aus Antikörpern, Fragmenten oder funktionellen Derivaten von Antikörpern und antikörperartigen Bindungsproteinen.

11. Zusammensetzung zur Verwendung gemäss irgendeinem der Ansprüche 8 bis 10, wobei die Polypeptide polyvalent sind und daher an mindestens zwei oder mehr Glykane binden.

12. Zusammensetzung zur Verwendung gemäss irgendeinem der Ansprüche 8 bis 11, wobei die Polypeptide ausgewählt sind aus der Gruppe der Pilzlektine, vorzugsweise Lektine aus *Coprinopsis cinerea, Xerocomus chrysenteron, Marasmius oreades, Aleuria aurantia* oder *Sordaria macrospora,* mehr bevorzugt Lektin CGL1, CGL2, RedA (CCL2), CCL1 XCL, MOA, AAL oder TAP1.

13. Zusammensetzung zur Verwendung gemäss irgendeinem der Ansprüche 8 bis 12, wobei die Glykan-bindenden Polypeptide auf der Oberfläche von Bakterienzellen dargestellt werden, vorzugsweise Enterobakterien, mehr bevorzugt menschliche Enterobakterien, am meisten bevorzugt *Escherichia coli* oder *Salmonella typhimurium.*

14. Nahrungsmittel umfassend isolierte Glykan-bindende Polypeptide gemäss irgendeinem der Ansprüche 1 bis 7 zur Verwendung in der Behandlung und/oder Vorbeugung einer helminthischen infektion, vorzugsweise einer Nernatodeninfektion.

15. Tierfutter umfassend isolierte Glykan-bindende Polypeptide gemäss irgendeinem der Ansprüche 1 bis 7 zur Verwendung in der Behandlung und/oder Vorbeugung einer helminthischen Infektion, vorzugsweise einer Nematodeninfektion.

## Revendications

1. Polypeptides de liaison à des glycanes à utiliser dans le traitement et/ou la prévention d'infections helminthiques, où les polypeptides de liaison à des glycanes se lient aux N-glycanes, O-glycanes ou lipoglycanes, où
I. les N-glycanes sont des oligo/polysaccharides et/ou des glycoconjugués contenant du fucoside, de préférence
(i) des oligo/polysaccharides et/ou des glycoconjugués contenant du D-galacto-pyranosyl-β-1,4-L-fucopyranosyle, plus préférentiellement des oligo/polysaccharides et/ou des glycoconjugués contenant du D-galacto-pyranosyl-β-1,4-L-fucopyranosyl-α-1,6-GlcNAc, plus préférentiellement des oligosaccharides et des glycoconjugués contenant du GnGnF⁶Gal et/ou du MMF⁶Gal, ou
(ii) des oligo/polysaccharides et/ou des glycoconjugués contenant du L-fucopyranosyl-α-1,3, plus préférentiellement des oligo/polysaccharides et/ou des glycoconjugués contenant du L-fucopyranosyl-α-1,3-GlcNAc, plus préférentiellement des oligosaccharides et des glycoconjugués contenant du GnGnF³ et/ou du MMF³;
II. les O-glycanes sont des oligo/polysaccharides et/ou des glycoconjugués contenant du galactoside, de préférence des oligo/polysaccharides et/ou des glycoconjugués contenant du D-galacto-pyranosyl-β-1,3, plus préférentiellement des oligo/polysaccharides et/ou des glycoconjugués contenant du D-galacto-pyranosyl-β-1,3-N-acétyl-D-galactosamino-pyranosyle, le plus préférentiellement des oligo/polysaccharides et/ou des glycoconjugués contenant du D-galacto-pyranosyl-β-1,3-N-acétyl-D-galactosamino-pyranosyl-O-Ser/Thr;
III. les lipoglycanes sont des oligo/polysaccharides et/ou des glycoconjugués contenant du galactoside, de préférence des oligo/polysaccharides et/ou des glycoconjugués contenant du D-galacto-pyranosyl-α-1,3, plus préférentiellement des oligo/polysaccharides et/ou des glycoconjugués contenant du D-galacto-pyranosyl-α-1,3-N-acétyl-D-galactosamino-pyranosyle, le plus préférentiellement des oligo/polysaccharides et/ou des glycoconjugués contenant du D-galacto-pyranosyl-α-1,3-N-acétyl-D-galactosamino-pyranosyl-β-1,4-N-acétyl-D-glucosamino-pyranosyle.

2. Polypeptides de liaison à des glycanes à utiliser selon la revendication 1, où les polypeptides se lient à au moins un glycane de nématodes, produit de préférence par des nématodes sélectionnés parmi la famille des Trichostrongylidae, de préférence *Haemonchus contortus, Trichostrongylus colubriformis, Teladorsagia circumcincta, Cooperia oncophora, Nematodirus battus, Ostertagia leptospicularis, Chabertia ovina, Oesophagostomum dentatum,* et les espèces de nématodes *Trichinella spiralis, Trichuris trichuria, Angiostrongylus vasorum, Ancylostoma caninum, Ancylostoma duodenale, Ancylostoma ceylanicum, Necator americanus*, *Dictyocaulus* spp., *Ascaris lumbricoides, Ascaris suum, Wuchereria bancrofti, Brugia malayi, Loa loa, Enterobius vermicularis, Dirofilaria immitis, Onchocerca volvulus* et *Strongyloides stercoralis.*

3. Polypeptides de liaison à des glycanes à utiliser selon l'une quelconque des revendications 1 ou 2, où les polypeptides sont sélectionnés parmi le groupe consistant en anticorps, fragments ou dérivés fonctionnels d'anticorps et protéines de liaison analogues à des anticorps.

4. Polypeptides de liaison à des glycanes à utiliser selon l'une quelconque des revendications 1 à 3, où les polypeptides sont polyvalents et se lient ainsi à au moins deux glycanes ou plus.

5. Polypeptides de liaison à des glycanes à utiliser selon les revendications 1 à 4, où les polypeptides de liaison à des glycanes sont sélectionnés parmi le groupe de lectines fongiques, de préférence des lectines de *Coprinopsis cinerea, Xerocomus chrysenteron, Marasmius oreades, Aleuria aurantia* ou *Sordaria macrospora,* plus préférentiellement des lectines sélectionnées parmi le groupe consistant en CGL2, CGL2, RedA (CCL2), CCL1, XCL, MOA, AAL et TAPI.

6. Polypeptides de liaison à des glycanes à utiliser selon l'une quelconque des revendications 1 à 5, où les polypeptides de liaison à des glycanes se lient à des N-glycanes et l'infection helminthique est une infection à nématodes.

7. Polypeptides de liaison à des glycanes à utiliser selon l'une quelconque des revendications 1 à 6, où l'infection helminthique, de préférence à nématodes, est une infection résultant d'un helminthe sélectionné parmi la famille des Trichostrongylidae, de préférence *Haemonchus contortus, Trichostrongylus colubriformis, Teladorsagia circumcincta, Cooperia oncophora, Nematodirus battus*, *Ostertagia leptospicularis, Chabertia ovina*, *Oesophagostomum dentatum,* et les espèces de nématodes *Trichinella spiralis*, *Trichuris trichuria, Angiostrongylus vasorum, Ancylostoma caninum, Ancylastoma duodenale, Ancylostoma ceylanicum, Necator americanus, Dictyocaulus* spp., *Ascaris lumbricoides*, *Ascaris suum, Wuchereria bancrofti, Brugia malayi, Loa loa, Enterobius vermicularis*, *Dirofilaria immitis, Onchocerca volvulus* et *Strongyloides stercoralis.*

8. Composition pharmaceutique comprenant au moins un polypeptide de liaison à des glycanes, à utiliser dans le traitement et/ou la prévention d'une infection helminthique, de préférence à nématodes, dans laquelle le polypeptide de liaison à des glycanes se lie à des N-glycanes, O-glycanes ou lipoglycanes, où
I. les N-glycanes sont des oligo/polysaccharides et/ou des glycoconjugués contenant du fucoside, de préférence
(i) des oligo/polysaccharides et/ou des glycoconjugués contenant du D-galacto-pyranosyl-β-1,4-L-fucopyranosyle, plus préférentiellement des oligo/polysaccharides et/ou des glycoconjugués contenant du D-galacto-pyranosyl-β-1,4-L-fucopyranosyl-α-1,6-GlcNAc, plus préférentiellement des oligosaccharides et des glycoconjugués contenant du GnGnF⁶Gal et/ou du MMF⁶Gal, ou
(ii) des oligo/polysaccharides et/ou des glycoconjugués contenant du L-fucopyranosyl-α-1,3, plus préférentiellement des oligo/polysaccharides et/ou des glycoconjugués contenant du L-fucopyranosyl-α-1,3-GlcNAc, plus préférentiellement des oligosaccharides et des glycoconjugués contenant du GnGnF³ et/ou du MMF³;
II. les O-glycanes sont des oligo/polysaccharides et/ou des glycoconjugués contenant du galactoside, de préférence des oligo/polysaccharides et/ou des glycoconjugués contenant du D-galacto-pyranosyl-β-1,3, plus préférentiellement des oligo/polysaccharides et/ou des glycoconjugués contenant du D-galacto-pyranosyl-β 1,3-N-acétyl-D-galactosamino-pyranosyle, le plus préférentiellement des 10 oligo/polysaccharides et/ou des glycoconjugués contenant du D-galacto-pyranosyl-β-1,3-N-acétyl-D-galactosamino-pyranosyl-O-Ser/Thr:
III. les lipoglycanes sont des oligo/polysaccharides et/ou des glycoconjugués contenant du galactoside, de préférence des oligo/polysaccharides et/ou des glycoconjugués contenant du D-galacto-pyranosyl-α-1,3, plus préférentiellement des 15 oligo/polysaccharides et/ou des glycoconjugués contenant du D-galacto-pyranosyl-α-1,3-N-acétyl-D-galactosamino-pyranosyle, le plus préférentiellement des oligo/polysaccharides et/ou des glycoconjugués contenant du D-galacto-pyranosyl-α-1,3 - N-αcétyl-D-galactosamino-pyranosyl-β-1,4-N-acétyl-D-glucosamino-pyranosyle.

9. Composition à utiliser selon la revendication 8, dans laquelle le polypeptide de 20 liaison à des glycanes se lient à au moins un glycane de nématodes produit par des nématodes sélectionné parmi la famille des Trichostrongylidae, de préférence *Haemonchus contortus, Trichostrongylus colubriformis, Teladorsagia circumcincta, Cooperia oncophora, Nematodirus battus, Ostertagia leptospicularis, Chabertia ovina, Oesophagostomum dentatum,* et les espèces de nématodes *Trichinella spiralis, Trichuris 25 trichuria, Angiostrongylus vasorum, Ancylostoma caninum, Ancylostoma duodenale, Ancylostoma ceylanicum, Necator americanus, Dictyocaulus* spp., *Ascaris lumbricoides*, *Ascaris suum, Wuchereria bancrofti, Brugia malayi, Loa loa, Enterobius vermicularis, Dirofilaria immitis, Onchocerca volvulus et Strongyloides stercoralis.*

10. Composition à utiliser selon l'une quelconque des revendications 8 ou 9, dans 30 laquelle les polypeptides sont sélectionnés parmi le groupe consistant en anticorps, fragments ou dérivés fonctionnels d'anticorps et protéines de liaison analogues à des anticorps.

11. Composition à utiliser selon l'une quelconque des revendications 8 à 10, dans laquelle les polypeptides sont polyvalents et se lient ainsi à au moins deux glycanes ou 35 plus.

12. Composition à utiliser selon l'une quelconque des revendications 8 à 11, dans laquelle les polypeptides sont sélectionnés parmi le groupe de lectines fongiques, de préférence des lectines de *Coprinopsis cinerea, Xerocomus chryserateron, Marasmius oreades, Aleuria aurantia* ou *Sordaria macrospora,* plus préférentiellement des lectines CGL1, CGL2, RedA (CCL2), CCL1, XCL, MOA, AAL ou TAP1.

13. Composition à utiliser selon l'une quelconque des revendications 8 à 12, dans laquelle les polypeptides de liaison à des glycanes se présentent à la surface de cellules bactériennes, de préférence des entérobactéries, plus préférentiellement des cellules entérobactériennes humaines, le plus préférentiellement *Escherichia coli* ou *Salmonella typhimurium.*

14. Produit alimentaire comprenant des polypeptides de liaison à des glycanes isolés selon l'une quelconque des revendications 1 à 7, à utiliser dans le traitement et/ou la prévention d'une infection helminthique, de préférence à nématodes.

15. Aliment pour animaux comprenant des polypeptides de liaison à des glycanes isolés selon l'une quelconque des revendications 1 à 7, à utiliser dans le traitement et/ou la prévention d'une infection helminthique, de préférence à nématodes.
